# EUROPEAN PATENT SPECIFICATION

(11) **EP 2 944 711 B1**
(45) Date of publication and mention of the grant of the patent: **27.12.2017**
(21) Application number: 14738104.0
(22) Date of filing: 10.01.2014
(51) Int. Cl.: D01F 1/10, A61K 9/70, A61K 33/26, A61K 41/00, A61K 47/32, A61P 35/00, C08F 220/56, D01D 5/04, D01F 6/28, D04H 1/413, D04H 1/728

(54) **NANOFIBER HAVING SELF-HEATING PROPERTIES AND BIOLOGICALLY ACTIVE SUBSTANCE RELEASE PROPERTIES, PRODUCTION METHOD FOR SAME, AND NONWOVEN FABRIC HAVING SELF-HEATING PROPERTIES AND BIOLOGICALLY ACTIVE SUBSTANCE RELEASE CAPABILITIES**
NANOFASER MIT SELBSTERWÄRMENDEN EIGENSCHAFTEN UND FREISETZUNG EINES BIOLOGISCHEN WIRKSTOFFES, HERSTELLUNGSVERFAHREN DAFÜR UND VLIESSTOFF MIT SELBSTERWÄRMENDEN EIGENSCHAFTEN SOWIE FREISETZUNG EINES BIOLOGISCHEN WIRKSTOFFES
NANOFIBRE AYANT DES PROPRIÉTÉS AUTOCHAUFFANTES ET DES PROPRIÉTÉS DE LIBÉRATION DE SUBSTANCE BIOLOGIQUEMENT ACTIVE, SON PROCÉDÉ DE PRODUCTION ET TISSU NON TISSÉ AYANT DES PROPRIÉTÉS AUTOCHAUFFANTES ET DES CAPACITÉS DE LIBÉRATION DE SUBSTANCE BIOLOGIQUEMENT ACTIVE

(30) Priority: 11.01.2013 JP 2013003341
(43) Date of publication of application: 18.11.2015
(73) Proprietor: National Institute for Materials Science, Tsukuba-shi, Ibaraki 305-0047 (JP)
(72) Inventor: EBARA, Mitsuhiro, Tsukuba-shi Ibaraki 305-0047 (JP); AOYAGI, Takao, Tsukuba-shi Ibaraki 305-0047 (JP); UTO, Koichiro, Tsukuba-shi Ibaraki 305-0047 (JP)
(74) Representative: V.O.
(86) International application number: PCT/JP2014/050306
(87) International publication number: WO 2014/109379

(56) References cited:
- WO-A1-2009/031523
- WO-A1-2013/032859
- WO-A2-2005/042142
- WO-A2-2007/092179
- JP-A- 2007 217 331
- JP-A- 2010 530 931
- YOUNG-JIN KIM ET AL.: 'Temperature-responsive electrospun nanofibers for 'on-off' switchable release of dextran' SCIENCE AND TECHNOLOGY OF ADVANCED MATERIALS vol. 13, no. 6, 18 October 2012, pages 1 - 9, XP055269401
- JAMES J. LAI ET AL.: 'Dual Magnetic-/Temperature-Responsive Nanoparticles for Microfluidic Separations and Assays' LANGMUIR vol. 23, no. ISSUE, 16 May 2007, pages 7385 - 7391, XP055006137

## Description

### Technical Field

The present invention relates to a nanofiber having self-heating properties and biologically active substance release properties, a production method for the same, and a nonwoven fabric having self-heating properties and biologically active substance release capabilities. Particularly, the invention relates to a nanofiber that has a function of self-heating in response to stimulation and releasing a biologically active substance so as to be used in a cancer thermochemotherapy treatment tool, a nonwoven fabric of the same, and a production method for the same.

### Background Art

A nanofiber is a fiber-shaped substance of which a diameter is several tens or several hundreds of nanometers, and of which a length is 100 times the diameter or longer, and is manufactured from one of nanomaterials. Since the nanofiber has a structure in which the specific surface area is extremely large compared with a general fiber, the nanofiber has unique physical characteristics in addition to existing characteristics of the polymer that a fiber in the related art has. For example, new functions such as excellent adsorption properties, excellent bonding properties, a hole control function in a nanometer order, a function derived from an advanced molecular structure, and excellent biocompatibility can be exhibited.

By utilizing these functions, a new subject material which did not exist in the related art can be developed, and thus the new subject material is expected to be used in a method that cannot be seen in the related art in many fields.

Such a nanofiber has received a great deal of attention in the biomedical field.

For example, a structure of a nanofiber structured body is similar to that of an extracellular matrix made of collagen or elastin, and thus characteristics required in a cell anchorage material for tissue construction such as porosity, dynamic intensity, and cell adhesion are satisfied. Therefore, the nanofiber structured body is applied to a regeneration treatment of bones, cartilage, blood vessels, or the like. In addition, since the specific surface area thereof is large, and handling thereof is easy, application to a wide range of areas such as a drug delivery system (DDS) or a medical material such as a synechia-preventive material is expected.

As a production method of a nanofiber, an electrospinning method, a melt-spinning method, a self-organizing method, a mold-synthesizing method, an electro-blowing method, a force-spinning method, and the like are widely used.

Among these, an electrospinning method has recently received attention since a nanofiber can be produced comparatively easily and on an industrial scale.

The electrospinning method is a method of performing spinning by extrafinely ejecting a polymer solution from a nozzle toward an electrode in a state in which a high voltage is applied between the nozzle and the electrode, evaporating a solvent from a flow of the extrafinely ejected polymer solution, and performing collection on the electrode. Nanofibers can be obtained in one step, and bulky nonwoven fabric can be manufactured with a small amount of raw material.

In addition, if various kinds of drugs are kneaded with the polymer solution, functionality can be easily applied to the nanofiber. For example, organic, inorganic, and metal composite nanofibers can be manufactured by dispersing gold nanoparticles, magnetic particles, carbon nanotubes, hydroxyapatite, or the like in the polymer solution.

Further, in the electrospinning method, precise structure control can be performed. For example, a sheath core (hollow) structured nanofiber can be manufactured by using a double pipe nozzle.

Manufacturing a conductive nanofiber or a stimulation-responsive nanofiber by using a polymer having its own function such as a conductive polymer or a stimulation-responsive polymer has been attempted.

The stimulation-responsive polymer is a polymer that sensitively responds to an external environmental change such as temperature, light, a magnetic field, an electric field, pH, or the like, and changes its physical properties. The stimulation-responsive polymer is also called a smart polymer.

As a temperature-responsive polymer, there is an N-alkyl-substituted acrylamide derivative polymer such as poly(N-isopropylacrylamide) (hereinafter, simply referred to as PNIPAAm).

PNIPAAm has been developed using a technique in which PNIPAAm is fixed to a biopolymer (protein, DNA, RNA, sugar chains, or the like), precipitated and dissolved just by a temperature change so that PNIPAAm is separated and collected, a technique in which PNIPAAm is introduced to a cell culture dish or a separation carrier surface, hydrophilicity or hydrophobicity of the surface is changed just by a temperature change, and a cell or a biologically active substance is collected, and the like.

As another type of temperature-responsive polymer, there is an aliphatic polyester derivative such as poly(ε-caprolactone) (hereinafter, simply referred to as PCL). PCL is a semicrystalline polymer, the crystallinity of which greatly changes on the border of the melting point, and thus transparency of the substance greatly changes near the melting point. Therefore, PCL has been developed to a transmission-controlling film of a drug (NPL 5). In addition, since PCL can maintain a shape obtained by deformation at the melting point or higher when being cooled to the melting point or lower, PCL can be applied to a shape memory material (NPL 6) or the like.

Recently, the inventors of the invention have developed a nanofiber of which characteristics drastically change in response to environmental temperature by using PNIPAAm and copolymers thereof.

In the related art, PNIPAAm is highly soluble in water and an organic solvent, and if a nanofiber made of PNIPAAm is synthesized, stability of the nanofiber in water is low, and the treatment of the nanofiber in water is difficult. In order to cause the treatment in water to be easy, a nanofiber which is chemically cross-linked is synthesized by using a polymer copolymerized with a monomer having a reactive group for cross-linking (NPL 1). The chemically cross-linked nanofiber is stable in water, and is easily treated in water. In addition, changes in structures and properties are reversibly repeated in response to temperature (NPL 2).

In addition, in the related art, if a temperature-responsive polymer is copolymerized with another monomer, a phase transition temperature thereof generally becomes unresponsive. However, if structures of copolymerized monomers are caused to be similar to each other, sensitive temperature responsiveness can be maintained (PTL 1).

Hyperthermia (thermal therapy for cancer) is one therapy for cancer, and is a therapy in which a section of a tumor is heated to 42°C to 43°C or higher and this temperature is maintained for 30 minutes to 60 minutes.

It can be expected that hyperthermia will enhance effects of a radiation therapy or a chemical therapy, and it is considered that the hyperthermia also has an effect of killing cancer cells (carcinostatic effect) because cancer cells are less resistant to heat than normal cells.

Recently, as clinically provided hyperthermia, a method of placing electrodes on the surface of a body, irradiating the surface of the body with radio waves, and increasing the temperature of the body of a patient by dielectric heating is used. In this method, since normal tissue between electrodes disposed on the surface of the body is also heated, the temperature of an area affected by cancer can be increased only to about 43°C so that the temperature of the normal tissue does not become too high. Accordingly, there is a problem in that the carcinostatic effect of the hyperthermia cannot be sufficiently exhibited.

As a method of selectively heating a tumor inside the body or a deep-seated tumor, magnetic hyperthermia has been developed. Magnetic hyperthermia uses a principle of applying an alternating magnetic field to the magnetic nanoparticles and self-heating magnetic nanoparticles generated by hysteresis loss due to magnetic wall movements (NPL 3). Only the area affected by the cancer can be selectively heated by collecting magnetic nanoparticles at the area affected by the cancer and causing the magnetic nanoparticles to self-heat using the principle above.

However, since nanoparticles such as magnetic nanoparticles diffuse into the human body, there is a concern that the nanoparticles may have a harmful effect on the human body (NPL 4). WO2005/042142 A discloses a polymer carrier in form of particles or fibres containing magnetic particles and active agents. The inductive heating of the magnetic particles leads to a change in physical structure of the polymer matrix and a liberation of the active agents.

### Citation List

### Patent Literature

PTL 1: Japanese Unexamined Patent Application, First Publication No. 2010-255001

### Non-Patent Literature

NPL 1: Y -J. Kim, M. Ebara, T. Aoyagi, Angew. Chem. Intl. Ed., 51, 10537 (2012)
NPL 2: Y -J. Kim, M. Ebara, T. Aoyagi, Sci. Technol. Adv. Mater., 13, 064202 (2012)
NPL 3: P. Techawanitchai, K. Yamamoto, M. Ebara, T. Aoyagi, Sci. Technol. Adv. Mater., 12, 044609 (2011)
NPL 4: V Kekkonen, N. Lafreniere, M. Ebara, A. Saito, Y Sawa, R. Narain, J. Magn. Magn. Mater., 321, 1393 (2009)
NPL 5: K. Uto, K. Yamamoto, S. Hirase, T. Aoyagi, J. Control. Rel., 110, 408 (2006)
NPL 6: M. Ebara, K. Uto, N. Idota, J. M. Hoffman, T. Aoyagi, Adv. Mater., 24, 273 (2012)

### Summary of Invention

### Technical Problem

An object of the invention is to provide a nanofiber having self-heating properties and biologically active substance release properties, which can release a drug at the same time as self-heating, and can be safely used on a human body without diffusing self-heating nanoparticles, a production method for the same, and a nonwoven fabric having self-heating properties and biologically active substance release capabilities.

### Solution to Problem

The inventors of the invention have developed a functional nanofiber and a nonwoven fabric that can self-heat and release a drug by embedding magnetic particles and a drug between polymers obtained by copolymerizing a temperature-responsive polymer and a monomer having a reactive group for cross-linking. The functional nanofiber and the nonwoven fabric cause the magnetic particles embedded therein to generate heat by applying an alternating magnetic field, and the temperature of the temperature-responsive polymer can be increased to the phase transition temperature or higher. In addition, the temperature-responsive polymer can be caused to thermally contract by increasing the temperature of the temperature-responsive polymer to the phase transition temperature or higher. In addition, a drug can be released to the outside together with water contained therein by causing the temperature-responsive polymer to thermally contract. Further, since the generation of heat by the magnetic particles is stopped by stopping the application of the alternating magnetic field, the temperature of the temperature-responsive polymer can decrease to the phase transition temperature or lower, and the size of the temperature-responsive polymer can expand back to the original size. If the alternating magnetic field is repeatedly applied and stopped, the shrinkage and the expansion of the temperature-responsive polymer are repeated with high reproducibility, and almost 100% of the drug included in the temperature-responsive polymer can be released to the outside. Based on this principle, in a test of disposing the nonwoven fabric on cancer cells and applying an alternating magnetic field, a thermal therapy by the generation of heat by the magnetic particles and a chemical therapy by the released drug can be applied to cancer cells at the same time, such that the cancer cells can be massively and effectively killed. According to the knowledge above, it has been found that the functional nanofiber and the nonwoven fabric can be used as thermochemotherapy treatment tools so as to complete the invention.

The invention has the following configurations.
(I) A fiber having a diameter in a range of 50 nm to 50 µm, and a length of 100 times the diameter or more, the fiber including: self-heating particles that generate heat in response to stimulation from outside; a stimulation-responsive polymer of which physical properties change by directly or indirectly reacting due to the stimulation; and a biologically active substance that is held by the stimulation-responsive polymer, in which the biologically active substance is released to the outside in response to the changes in the physical properties of the stimulation-responsive polymer.
(II) The fiber according to (I), further including water.
(III) The fiber according to (I) or (II), in which the stimulation-responsive polymer includes a structure in which a plurality of polymer cross-linked bodies are cross-linked to each other.
(IV) The fiber according to any one of (I) to (III), in which a diameter of the fiber is 50 nm or longer and shorter than 1 µm.
(V) The fiber according to any one of (I) to (IV), in which the self-heating particles are any one of magnetic particles, gold nanorods, gold particles, and carbon nanotubes, or a combination thereof.
(VI) The fiber according to any one of (V), in which the self-heating particles are magnetic particles made of iron oxide.
(VII) The fiber according to any one of (V) to (VI),
   wherein a particle diameter of the self-heating particle is in a range of 10 nm to 10 µm.
(VIII) The fiber according to any one of (1) to (VII), in which a weight ratio of the self-heating particles is in a range of 10 wt% to 50 wt% with respect to a total weight of the fiber.
(IX) The fiber according to any one of (I) to (VIII), in which the biologically active substance is a particle which contains an anticancer drug.
(X) The fiber according to (IX), in which the particle diameter of the particles is 10 nm or shorter.
(XI) The fiber according to (IX) or (X), in which a weight ratio of the particles is in a range of 0.1 wt% to 10 wt% with respect to a total weight of the fiber.
(XII) The fiber according to any one of (I) to (XI), in which the stimulation-responsive polymer is any one selected from the group consisting of a temperature-responsive polymer, a light-responsive polymer, a magnetic field-responsive polymer, an electric field-responsive polymer, and a pH-responsive polymer.
(XIII) The fiber according to (XII), in which the temperature-responsive polymer has a polyethylene main chain and an N-alkyl-substituted acrylamide side chain.
(XIV) The fiber according to (XIII), further including a polymer cross-linked body represented by Formula (1) below, in which, in a polymer cross-linked body, a polymer having a polyethylene main chain R₁(CH₂CH)ₗ(CH₂CH)ₘR₃ and an N-alkyl-substituted acrylamide side chain CONHR₂, and a thermally- or photo-crosslinkable substituent X and a polymer having a polyethylene main chain R₄(CH₂CH)ₛ(CH₂CH)ₜR₅, an N-alkyl-substituted acrylamide side chain CONHR₂, and a thermally- or photo-crosslinkable substituent X are cross-linked with the thermally- or photo-crosslinkable substituents X, so that a cross-linked portion X...X is formed.
   (In Formula (1) below, substituents R₁, R₃, R₄, and R₅ are hydrogen atoms or linear or branched alkyl groups having 1 to 6 carbon atoms, and a substituent R₂ is any alkyl group selected from the group consisting of an isopropyl group, an n-propyl group, and a butylacrylamide. In addition, 1, m, s, and t respectively represent molar ratios (%) of monomers, and a sum of 1 and m and a sum of s and t are 100%.) 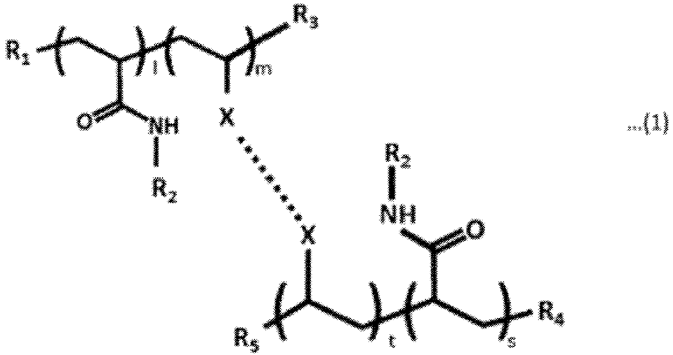
(XV) The fiber according to (XIV), in which the cross-linked portion X...X is represented by Formula (2) below.
   (In Formula (2) below, n is a natural number in a range of 1 to 6. A is an NH group and a linking group of O.) 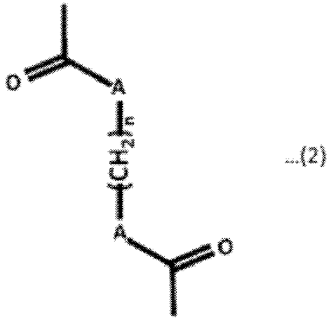
(XVI) A production method for a fiber including: a step of synthesizing a first polymer which is a stimulation-responsive polymer including a polyethylene main chain, a stimulation-responsive side chain, and a thermally- or photo-crosslinkable side chain by dispersing a first monomer having a stimulation-responsive functional group, a second monomer having a thermally- or photo-crosslinkable functional group, and a polymerization initiator in a solvent, and performing copolymerization by heat in a degassed atmosphere; a step of preparing a first polymer solution by dispersing the first polymer, self-heating particles, and a biologically active substance in a solvent; a step of producing a stimulation-responsive fiber containing the self-heating particles and the biologically active substance by spinning the first polymer solution by an electrospinning method; and a step of producing a fiber made of a cross-linked body of the stimulation-responsive fiber containing the self-heating particles and the biologically active substance by cross-linking a stimulation-responsive fiber containing the self-heating particles and the biologically active substance by heat or light.
(XVII) The production method for a fiber according to (XVI), in which the self-heating particles are magnetic particles.
(XVIII) The production method for a fiber according to (XVI) or (XVII), in which the first monomer is a monomer including the stimulation-responsive functional group.
(XIX) The production method for a fiber according to (XVIII), in which the first monomer is a monomer of an N-alkyl-substituted acrylamide derivative represented by Formula (3) below.
   (In Formula (3) below, an N-alkyl-substituted acrylamide group which is a stimulation-responsive functional group is included, a substituent R₁ is a hydrogen atom or a linear or branched alkyl group having 1 to 6 carbon atoms, and a substituent R₂ is any alkyl group selected from the group consisting of an isopropyl group, an n-propyl group, and a butylacrylamide.) 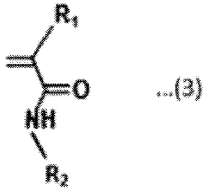
(XX) The production method for a fiber according to any one of (XVI) to (XIX), in which the second monomer is a cross-linking monomer having a thermally- or photo-crosslinkable functional group.
(XXI) The production method for a fiber according to (XX), in which the second monomer is a monomer represented by Formula (4) below.
   (In Formula (4) below, a substituent R₃ is a hydrogen atom or a linear or branched alkyl group having 1 to 6 carbon atoms, and a substituent X is a thermally- or photo-crosslinkable functional group represented by Formula (5) below.
   In Formula (5) below, n is a natural number in a range of 1 to 6. A is an NH group or a linking group of O.) 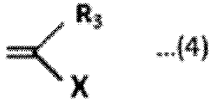 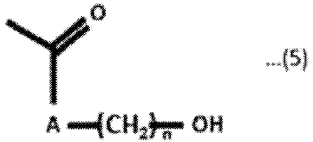
(XXII) The production method for a fiber according to (XVI), in which a condition of the electrospinning method is a flow velocity in a range of 0.1 mL/h to 10 mL/h, and a voltage in a range of 10 kV to 50 kV.
(XXIII) The production method for a fiber according to (XVI), in which, in the step of producing the fiber, the heating is performed under the condition of a temperature in a range of 100°C to 150°C and a period of time in a range of 10 hours to 20 hours.
(XXIV) A nonwoven fabric, in which the fibers according to any one of (I) to (XV) are bonded to each other in a mesh shape to form a sheet shape.

In addition, another aspect of the invention provides the following aspects.
(1) A nanofiber having self-heating/drug release capabilities, of which a diameter is 50 nm or longer and shorter than 1 µm, in which a stimulation-responsive polymer is formed of a plurality of polymer cross-linked bodies which are cross-linked to each other, and self-heating particles, drug particles, and water are contained between stimulation-responsive polymers.
(2) The nanofiber having self-heating/drug release capabilities according to (1), in which the self-heating particles are any one of magnetic particles, gold nanorods, gold particles, and carbon nanotubes, or a combination thereof.
(3) The nanofiber having self-heating/drug release capabilities according to (2), in which the self-heating particles are magnetic particles made of iron oxide.
(4) The nanofiber having self-heating/drug release capabilities according to (2) or (3), in which a particle diameter of the self-heating particle is in a range of 10 nm to 100 nm.
(5) The nanofiber having self-heating/drug release capabilities according to any one of (1) to (4), in which the concentration of the self-heating particles is in a range of 10 wt% to 50 wt%.
(6) The nanofiber having self-heating/drug release capabilities according to any one of (1) to (5), in which the drug particles form an anticancer drug.
(7) The nanofiber having self-heating/drug release capabilities according to (6), in which the particle diameter of the drug particles is 10 nm or shorter.
(8) The nanofiber having self-heating/drug release capabilities according to (6) or (7), in which the concentration of the drug particles is in a range of 0.1 wt% to 1 wt%.
(9) The nanofiber having self-heating/drug release capabilities according to any one of (1) to (8), in which the stimulation-responsive polymer is any one selected from the group consisting of a temperature-responsive polymer, a light-responsive polymer, a magnetic field-responsive polymer, an electric field-responsive polymer, and a pH-responsive polymer.
(10) The nanofiber having self-heating/drug release capabilities according to (9), in which the temperature-responsive polymer has a polyethylene main chain and an N-alkyl-substituted acrylamide side chain.
(11) The nanofiber having self-heating/drug release capabilities according to (10), further including: a polymer cross-linked body represented by Formula (1) below, in which, in a polymer cross-linked body, a polymer having a polyethylene main chain R₁(CH₂CH)₁(CH₂CH)ₘR₃ and an N-alkyl-substituted acrylamide side chain CONHR₂, and a thermally- or photo-crosslinkable substituent X and a polymer having a polyethylene main chain R₄(CH₂CH)ₛ(CH₂CH)ₜR₅, an N-alkyl-substituted acrylamide side chain CONHR₂, and a thermally- or photo-crosslinkable substituent X are cross-linked with the thermally- or photo-crosslinkable substituents X, so that a cross-linked portion X...X is formed. In Formula (1) above, substituents R₁, R₃, R₄, and R₅ are hydrogen atoms or linear or branched alkyl groups having 1 to 6 carbon atoms, and a substituent R₂ is any alkyl group selected from the group consisting of an isopropyl group, an n-propyl group, and a butylacrylamide. In addition, 1, m, s, and t respectively represent molar ratios (%) of monomers, and a sum of 1 and m and a sum of s and t are 100%.
(12) The nanofiber having self-heating/drug release capabilities according to (11), in which the cross-linked portion X...X is represented by Formula (2) below. In Formula (2) above, n is a natural number in a range of 1 to 6. A is an NH group and a linking group of O.
(13) A production method for a nanofiber having self-heating/drug release capabilities including: a step of synthesizing a first polymer which is a stimulation-responsive polymer including a polyethylene main chain, a stimulation-responsive side chain, and a thermally- or photo-crosslinkable side chain by dispersing a first monomer having a stimulation-responsive functional group, a second monomer having a thermally- or photo-crosslinkable functional group, and a polymerization initiator in a solvent, and performing copolymerization by heat in a degassed atmosphere; a step of preparing a first polymer solution by dispersing the first polymer, self-heating particles, and drug particles in a solvent; a step of producing a stimulation-responsive nanofiber containing the self-heating particles and the drug particles by spinning the first polymer solution by an electrospinning method; and a step of producing a nanofiber having self-heating/drug release capabilities made of a cross-linked body of the stimulation-responsive fiber containing the self-heating particles and the drug particles by cross-linking a stimulation-responsive fiber containing the self-heating particles and the drug particles by heat or light.
(14) The production method for a nanofiber having self-heating/drug release capabilities according to (13), in which the self-heating particles are magnetic particles.
(15) The production method for a nanofiber having self-heating/drug release capabilities according to (13) or (14), in which the first monomer is a monomer including the stimulation-responsive functional group.
(16) The production method for a nanofiber having self-heating/drug release capabilities according to (15), in which the first monomer is a monomer of an N-alkyl-substituted acrylamide derivative represented by Formula (3) below.
   In Formula (3) above, an N-alkyl-substituted acrylamide group which is a stimulation-responsive functional group is included, a substituent R₁ is a hydrogen atom or a linear or branched alkyl group having 1 to 6 carbon atoms, and a substituent R₂ is any alkyl group selected from the group consisting of an isopropyl group, an n-propyl group, and a butylacrylamide.
(17) The production method for a nanofiber having self-heating/drug release capabilities according to any one of (13) to (16), in which the second monomer is a cross-linking monomer having a thermally- or photo-crosslinkable functional group.
(18) The production method for a nanofiber having self-heating/drug release capabilities according to (17), in which the second monomer is a monomer represented by Formula (4) below. In Formula (4) above, a substituent R₃ is a hydrogen atom or a linear or branched alkyl group having 1 to 6 carbon atoms, and a substituent X is a thermally- or photo-crosslinkable functional group represented by Formula (5) above. In Formula (5) above, n is a natural number in a range of 1 to 6. A is an NH group or a linking group of O.
(19) The production method for a nanofiber having self-heating/drug release capabilities according to (13), in which a condition of the electrospinning method is a flow velocity in a range of 0.1 mL/h to 10 mL/h, and a voltage in a range of 10 kV to 50 kV.
(20) The production method for a nanofiber having self-heating/drug release capabilities according to (13), in which, in the step of producing the nanofiber having self-heating/drug release capabilities, the heating is performed under the condition of a temperature in a range of 100°C to 150°C and a period of time in a range of 10 hours to 20 hours.
(21) A nonwoven fabric, in which the nanofibers having self-heating/drug release capabilities according to any one of (1) to (12) are bonded to each other in a mesh shape to form a sheet shape.

### Advantageous Effects of Invention

According to an aspect of the invention, a fiber is provided having a diameter in a range of 50 nm to 50 µm, and a length of 100 times the diameter or more and including: self-heating particles that generate heat in response to stimulation from outside; a stimulation-responsive polymer that directly or indirectly reacts with the stimulation and of which physical properties change; and a biologically active substance that is held by the stimulation-responsive polymer, in which the biologically active substance is released to the outside in response to the changes in the physical properties of the stimulation-responsive polymer. According to the effect of the configuration, the fiber self-heats, and at the same time, the biologically active substance can be released. The thermal therapy can be applied to an affected area by disposing the fibers on the affected area and causing the self-heating particles in the fiber to self-heat by the stimulation from outside of the fiber. In addition, in response to direct or indirect stimulation from outside, physical properties of the stimulation-responsive polymer change, the inside biologically active substance can be released to the outside, and thus chemical therapy by a drug can be applied to the affected area. Also, the self-heating particles are stably included in the fiber, and thus even if the physical properties of the fiber change, the self-heating particles do not diffuse to the outside together with the biologically active substance such that the fiber can be safely used on the human body.

According to another aspect of the invention, a nanofiber is provided having self-heating/drug release capabilities of which a diameter is 50 nm or longer and shorter than 1 µm, in which the stimulation-responsive polymer is formed of a plurality of polymer cross-linked bodies which are cross-linked to each other, and self-heating particles, drug particles, and water are contained between stimulation-responsive polymers, and thus the nanofiber can self-heat and can release a drug at the same time. Particularly, since magnetic particles are used as the self-heating particles, the nanofiber can be caused to generate heat by disposing a nanofiber on an affected area, irradiating the affected area with an alternating magnetic field, and causing the inside self-heating particles to self-heat such that thermal therapy can be applied to the affected area. In addition, since the temperature-responsive polymer is used as the stimulation-responsive polymer, the temperature-responsive fiber contracts in response to the heat generated by self-heating, the inside drug particles can be released to the outside together with inside water, and thus chemical therapy due to the drug can be applied to the affected area. In addition, since an anticancer drug is used as the drug, apoptosis of the cancer cells can be promoted. In addition, since magnetic particles are used as the self-heating particles, positions and directions of the nanofiber disposed on the body can be manipulated by using a magnet from outside. Further, the self-heating particles are stably included in the nanofiber, and thus even if the nanofiber contracts, the self-heating nanoparticles do not diffuse to the outside together with water and the drug particles such that the fiber can be safely used on the human body.

According to an aspect of the invention, a production method for a fiber is provided including a step of synthesizing a first polymer which is a stimulation-responsive polymer including a polyethylene main chain, a stimulation-responsive side chain, and a thermally- or photo-crosslinkable side chain by dispersing a first monomer having a stimulation-responsive functional group, a second monomer having a thermally- or photo-crosslinkable functional group, and a polymerization initiator in a solvent, performing copolymerization by heat in a degassed atmosphere; a step of preparing a first polymer solution by dispersing the first polymer, self-heating particles, and a biologically active substance in a solvent; a step of producing a stimulation-responsive fiber containing the self-heating particles and the drug particles by spinning the first polymer solution by an electrospinning method; and a step of producing a fiber made of a cross-linked body of the stimulation-responsive fiber containing the self-heating particles and the biologically active substance by cross-linking a stimulation-responsive fiber containing the self-heating particles and the biologically active substance by heat or light. Therefore, a fiber that self-heats in response to the environment, that can release a biologically active substance, in which self-heating particles are stably included in the fiber by cross-linking, that does not diffuse self-heating particles to the outside together with a biologically active substance even if physical properties of the fiber change, and that can be safely used on the human body can be easily produced.

According to another aspect of the invention, a production method is provided for a nanofiber having self-heating/drug release capabilities including a step of synthesizing a first polymer which is a stimulation-responsive polymer including a polyethylene main chain, a stimulation-responsive side chain, and a thermally- or photo-crosslinkable side chain by dispersing a first monomer having a stimulation-responsive functional group, a second monomer having a thermally- or photo-crosslinkable functional group, and a polymerization initiator in a solvent, and performing copolymerization by heat in a degassed atmosphere; a step of preparing a first polymer solution by dispersing the first polymer, self-heating particles, and drug particles in a solvent; a step of producing a stimulation-responsive nanofiber containing the self-heating particles and the biologically active substance by spinning the first polymer solution by an electrospinning method; and a step of producing a nanofiber, having self-heating/drug release capabilities, which is made of a cross-linked body of the stimulation-responsive nanofiber containing the self-heating particles and the drug particles by cross-linking the stimulation-responsive nanofiber containing the self-heating particles and the drug particles by heat or light. Therefore, a nanofiber having self-heating/drug release capabilities, that self-heats in response to the environment, and that can release a drug, in which self-heating particles are stably included in the fiber by cross-linking, that does not diffuse self-heating nanoparticles to the outside together with water and the drug particles even if the nanofiber contracts, and that can be safely used on the human body can be easily produced.

According to an aspect of the invention, a nonwoven fabric is provided (according to another aspect of the invention, a nonwoven fabric having self-heating/drug release capabilities) in which the fibers or the nanofibers having self-heating/drug release capabilities are bonded in a mesh shape to form a sheet shape. Therefore, a shape in planar view can be easily processed according to a shape of the affected area.

### Brief Description of Drawings

FIG. 1A is a planar view illustrating an example of a nonwoven fabric according to an embodiment.
FIG. 1B is a front view illustrating an example of the nonwoven fabric according to the embodiment.
FIG. 2 is an enlarged view of a portion A in FIG. 1A.
FIG. 3 is an enlarged view of a portion B in FIG. 2, and illustrating an example of a fiber according to the embodiment.
FIG. 4 is a flow chart illustrating an example of releasing a drug of the fiber according to the embodiment.
FIG. 5 is an explanation diagram illustrating an appearance of releasing a drug in a drug-releasing step S4 of FIG. 4.
FIG. 6 is a flow chart illustrating an example of a production method for the fiber according to the embodiment.
FIG. 7 is a drawing illustrating an example of a cancer treatment performed by using a nonwoven fabric according to the embodiment.
FIG. 8 is a graph illustrating a 1H-NMR spectrum measurement result of a sample in Test Example 1.
FIG. 9 is a graph illustrating environmental temperature dependency of a UV spectrum measurement result.
FIG. 10 is a scanning electron microscope (SEM) picture of a sample (NIPAAm-HMAAm copolymer fiber) in Test Example 2.
FIG. 11 is a scanning electron microscope (SEM) picture of a sample (NIPAAm-HMAAm copolymer fiber containing magnetic particles and anticancer drug) in Test Example 3.
FIG. 12 is a scanning electron microscope (SEM) picture of a sample (NIPAAm-HMAAm copolymer fiber containing magnetic particles and anticancer drug after cross-linking) in Example 1.
FIG. 13 is a transmission electron microscope (TEM) picture of a sample (NIPAAm-HMAAm copolymer fiber containing magnetic particles and anticancer drug after cross-linking) in Example 1.
FIG. 14 is an XRD measurement result of the NIPAAm-HMAAm copolymer fiber containing magnetic particles and an anticancer drug before or after cross-linking. FIG. 14 is a comparison of the sample in Example 1 and the sample in Test Example 3.
FIG. 15A is a graph illustrating heat generation behavior of the sample in Example 1, and is a graph illustrating a relationship between an alternating magnetic field irradiation time and surface temperature of the sample.
FIG. 15B is a graph illustrating heat generation behavior of the sample in Example 1 and is a graph illustrating a relationship between an alternating magnetic field irradiation time and surface temperature of the sample.
FIG. 16 is a diagram including pictures illustrating differences between alternating magnetic field irradiation times of observation images using an infrared camera.
FIG. 17 is a graph illustrating a relationship of surface temperature of the sample with respect to switching of alternating magnetic fields and an explanation diagram (upper diagram) thereof.
FIG. 18 is a diagram including pictures illustrating changes of positions of fibers according to the elapsed time when a neodymium magnet is brought close.
FIG. 19 is a graph illustrating expansion and contraction behavior of the sample in Example 1.
FIG. 20 is a diagram including atomic force microscope (AFM) images of the sample in Example 1 in water, in which FIG. 20(a) is an image at 25°C, and FIG. 20(b) is an image at 45°C.
FIG. 21 is a graph illustrating relationships between expansion and contraction behaviors in response to alternating magnetic fields and release of a drug with respect to the sample in Example 1.
FIG. 22 is a graph illustrating a relationship between cell culture periods and cell proliferation indexes.
FIG 23 is a diagram including microscope observation pictures of respective samples at time points of fifth days in a cell culture period.
FIG. 24 is a graph illustrating a 1H-NMR spectrum measurement result of a sample in Test Example 4.
FIG. 25 is a scanning electron microscope (SEM) image of a sample in Test Example 5.
FIG. 26 is a diagram including pictures illustrating differences between alternating magnetic field irradiation times of observation images of the infrared camera with respect to the sample in Test Example 5.

### Description of Embodiments

### (Embodiment of the invention)

Hereinafter, with reference to the accompanied drawings, a fiber and a nonwoven fabric according to an embodiment of the invention are described.

### <Nonwoven fabric>

A fiber according to an embodiment can be used in a form of a subject material using the fiber, for example, a subject material formed by knitting or bonding the fibers, but in the embodiment, a product in a form of a nonwoven fabric using a fiber as a subject material is used. Hereinafter, a nonwoven fabric according to the embodiment (nonwoven fabric having self-heating properties and biologically active substance release capabilities or nonwoven fabric having self-heating/drug release capabilities) is described.

Here, the nonwoven fabric is a cloth-form subject material produced without a step of intertwining or knitting a linear constituent material (fiber according to the embodiment), and is a subject material mainly having a structure in which the constituent material is entangled.

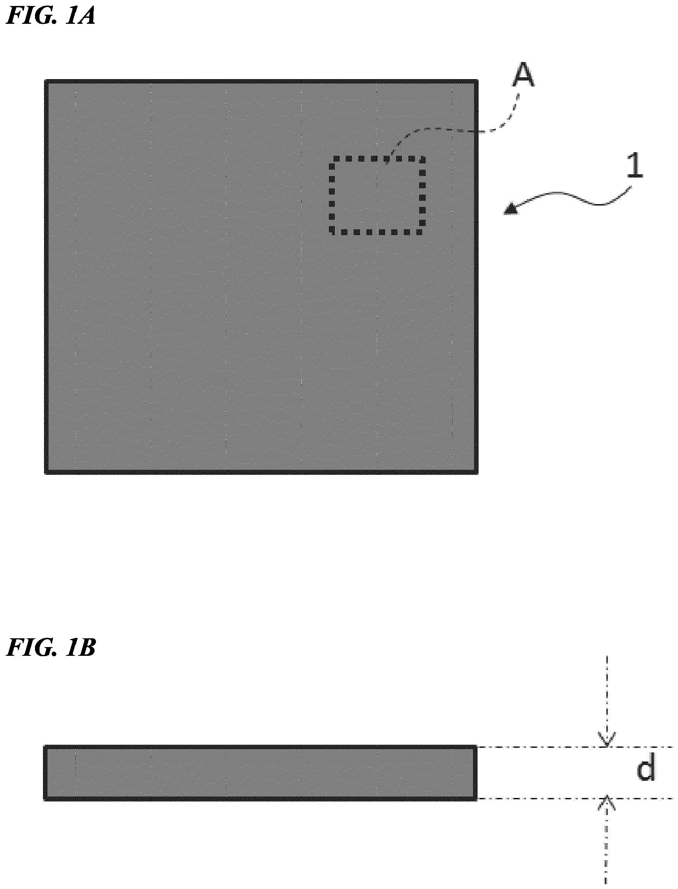

FIGS. 1A and 1B are diagrams illustrating an example of the nonwoven fabric according to the embodiment, in which FIG. 1A is a planar view and FIG. 1B is a front view.

In FIG. 1A, a nonwoven fabric 1 according to an embodiment has an approximately rectangular shape. However, the invention is not limited to this shape, and may have a circular shape, a polygonal shape, or the like.

As illustrated in FIGS. 1A and 1B, the nonwoven fabric 1 according to the embodiment has a sheet shape. Accordingly, a shape in planar view can be easily processed according to a shape of an affected area, such that the nonwoven fabric 1 can be bonded according to the shape of the affected area and thermal therapy and drug therapy can be effectively applied.

In addition, a thickness d of the nonwoven fabric 1 according to the embodiment is preferably in a range of 1 µm to 1 mm. Therefore, the shape in planar view can be easily processed according to the shape and the size of the affected area such that the nonwoven fabric 1 can be adhesively bonded according to the shape of the affected area.

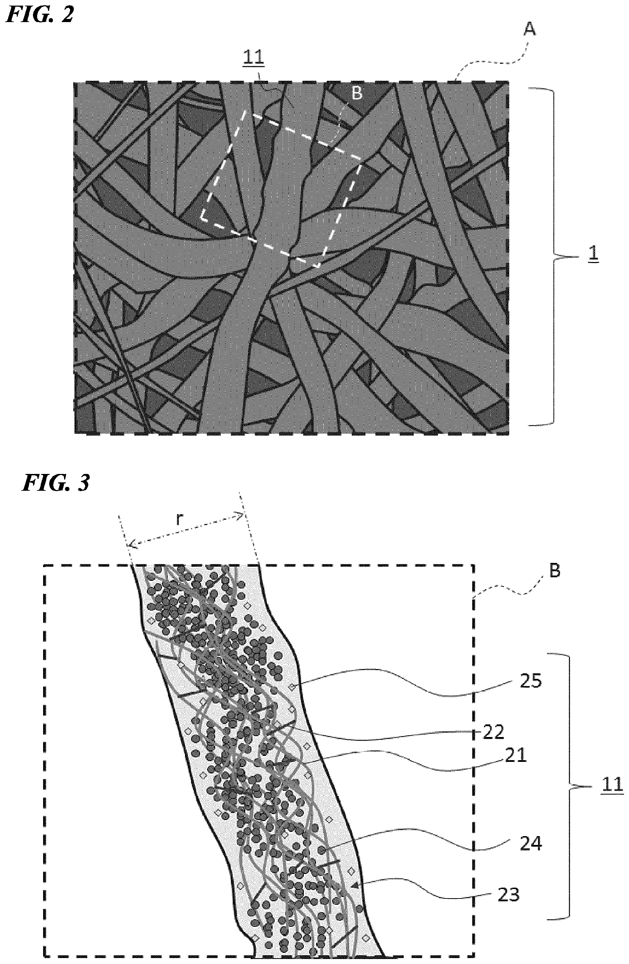

FIG. 2 is an enlarged view of a portion A in FIG. 1A.

As illustrated in FIG. 2, the nonwoven fabric 1 according to the embodiment is schematically formed by causing fibers 11 to be in a mesh shape. The fiber 11 according to the embodiment may be called a nanofiber having self-heating/drug release capabilities if the diameter of the fiber is in a nanometer order. In addition, the fibers 11 are partially bonded to each other.

### <Fiber>

Next, the fiber according to the embodiment is described.

As illustrated in FIG. 3, the fiber 11 according to the embodiment includes a stimulation-responsive polymer 21, self-heating particles 24, and a biologically active substance held by the stimulation-responsive polymer 21.

FIG. 3 is an enlarged view of a portion B in FIG. 2, and illustrating an example of a fiber according to the embodiment.

The fiber 11 according to the embodiment is formed by causing plural stimulation-responsive polymers 21 to be entangled. According to the embodiment, the stimulation-responsive polymers 21 contain polymer cross-linked bodies which are cross-linked in cross-linked portions 22. According to the embodiment, the self-heating particles 24, the biologically active substance (drug particles 25 according to embodiment), and water 23 are contained between the stimulation-responsive polymers 21.

The fiber according to the embodiment is a thin and long material, and is a material that can form a subject material such as cloth or thread by being entangled with each other. The fiber according to the embodiment has a diameter in a range of 50 nm to 50,000 nm (50 µm), as a standard, and a length which is 100 times the diameter, as a standard.

In addition, the fiber 11 according to the embodiment preferably has a diameter r which is 50 nm or longer and shorter than 1 µm (1,000 nm). The fibers that have a diameter in this range and many of which are measured in the units of nm are particularly called nanofibers. The diameter r is preferably in a range of 100 nm to 800 nm, and more preferably in a range of 200 nm to 500 nm.

### [Self-heating particle]

With respect to the self-heating particles according to the embodiment, the expression "self-heating" refers to increasing temperature (that is, generating heat) in response to stimulation from outside. The expression "from outside" refers to stimulation by a cause other than the particle, mainly applied from outside of the particle. The stimulation includes electromagnetic waves such as heat or light, magnetic fields, or physical stimulation (vibration, impact, or the like). As described below, the particle is a part of a substance of which the diameter is less than a millimeter in size as described below, and according to the embodiment, particularly ones of which the diameter is in a range of 1 nm to 1,000 nm.

The self-heating particles 24 are preferably any one of magnetic particles, gold nanorods, gold particles, and carbon nanotubes, or a combination thereof. It is possible to effectively cause the fiber to self-heat, by combining materials.

Particularly, the self-heating particles 24 are preferably magnetic particles made of iron oxide. In this case, the self-heating particles 24 can effectively self-heat by being irradiated with an alternating magnetic field. In addition, the positions and the directions of the fiber can be manipulated by the magnetic field.

If the gold nanorods, the gold particles, and the carbon nanotubes are used, the fiber can be caused to self-heat, by using methods such as near infrared light irradiation.

The self-heating particles 24 preferably have a particle diameter in a range of 10 nm to 10 µm. The polymer cross-linked body has a three-dimensional network structure having pouch-shaped spaces in various sizes therein, and forms a hydrogel due to water being contained. If the particle diameter is 10 nm or longer, the self-heating particles 24 that are included in the spaces and are entangled into polymer chains are not easily released to the outside even if the polymer cross-linked body contracts. In addition, a predetermined self-heating function can be performed. Further, if the magnetic particles are used as the self-heating particles 24, the fiber can be manipulated by a magnet by accumulating the magnetic particles in the polymer chain.

If the particle diameter is 10 nm or shorter, when the polymer cross-linked body contracts, the self-heating particles may be leaked to the outside from portions between the polymer chains. In addition, if the particle diameter is 10 µm or longer, it may be difficult to disperse the self-heating particles between the polymer chains. In order to satisfactorily disperse the self-heating particles 24 between the polymer chains, the particle diameter is preferably 100 nm or shorter. When the magnetic particles are used as the self-heating particles 24, if the particles are not accumulated in the polymer chain, the fiber may not be manipulated by a magnet.

The weight ratio (or concentration) of the self-heating particles 24 to the total weight is preferably in a range of 10 wt% to 50 wt%. In this case, the self-heating particles can effectively self-heat. Further, if magnetic particles are used as the self-heating particles 24, the fiber can be manipulated by the magnet.

The form in which the self-heating particles 24 are included in the fiber 11 is not particularly limited, but according to the embodiment, the self-heating particles 24 are held by the stimulation-responsive polymer 21. The expression "hold" refers to keeping a state of being close to the stimulation-responsive polymer 21 if there is no external stimulation. For example, a state of being adhered to the stimulation-responsive polymer 21 without being chemically bonded to the outside or a portion near the outside of the polymer, a state of being adhered by being entangled with or interposed between the stimulation-responsive polymers 21, a state of being embedded in the polymer to a degree in which the particles can be detached from a polymer by physical force, or a state of being adhered to a portion between molecules in the structure of the fiber 11 containing the stimulation-responsive polymer 21 are included.

According to the embodiment, as illustrated in FIG. 3, the self-heating particles 24 include products in which the self-heating particles 24 are interposed or entangled between polymers of the stimulation-responsive polymer 21, and products in which the self-heating particles 24 are held in a state of being adhered to portions between molecules in the structure of the fiber 11 made of the stimulation-responsive polymer 21 and the water 23.

### [Biologically active substance]

The fiber 11 according to the embodiment contains a biologically active substance. The biologically active substance refers to a substance having influence on an organism, particularly, a substance that exhibits activity in a physiological function.

According to the embodiment, as the biologically active substance, drug particles are used. The drug refers to a biologically active substance that is mainly used for treatments or prevention of diseases, or the like. The drug particles refer to products having the drug in a form of particles having a particle diameter described below. As the drug particles 25, various kinds of drugs for various diseases are included, and according to the embodiment, particles particularly made of an anticancer drug or containing an anticancer drug are included. In addition, according to an aspect of the fiber according to the embodiment, a nano-sized fiber containing drug particles as a biologically active substance may be called a nanofiber having self-heating/drug release capabilities.

If the drug 25 particles made of an anticancer drug are used as the biologically active substance, and the fiber 11 according to the embodiment and/or the nonwoven fabric is disposed on a cancer cell, a thermal therapy and chemotherapy by an anticancer drug can be performed at the same time.

The particle diameter of the drug particles 25 is preferably 10 nm or shorter. If the particle diameter is 10 nm or shorter, when the fiber 11 contracts, the drug particles can be easily released to the outside together with water. If the particle diameter is longer than 10 nm, when the fiber 11 contracts, the release may be difficult.

The weight ratio (concentration) of the drug particles 25 to the total weight of the fiber 11 is preferably in a range of 0.1 wt% to 10 wt%. If the weight ratio is 0.1 wt% or greater, when the fiber 11 contracts, a sufficient amount of the drug particles 25 can be released to the outside, so that the drug effect can be held to a certain degree or more.

When the weight ratio is greater than 10 wt%, excessive drug may be released. Further, the more sufficient releasing amount of the drug may be 1 wt% or less.

The biologically active substance is held by the stimulation-responsive polymer 21. The expression "hold" refers to keeping a state of being close to the stimulation-responsive polymer 21 if there is no external stimulation. For example, a state of being adhered to the stimulation-responsive polymer 21 without being chemically bonded to the outside or a portion near the outside of the polymer, a state of being adhered by being entangled with or interposed between the stimulation-responsive polymers 21, a state of being embedded in the polymer in a degree in which the particles can be detached from a polymer by physical force, or a state of being adhered to a portion between molecules in the structure of the fiber 11 containing the stimulation-responsive polymer 21 are included.

According to the embodiment, as illustrated in FIG. 3, the drug particles 25 are held in a state of being adhered to portions between molecules in the structure of the fiber 11 made of the stimulation-responsive polymer 21 and the water 23.

### [Stimulation-responsive polymer]

The stimulation-responsive polymer 21 is a polymer that directly or indirectly reacts to the stimulation caused by the change of the external environment and of which physical properties (physicality) are changed.

Specifically, the stimulation-responsive polymer 21 is any one selected from the group consisting of the temperature-responsive polymer responding to the environmental temperature change, a light-responsive polymer responding to the environmental light change, a magnetic field-responsive polymer responding to the environmental magnetic field change, an electric field-responsive polymer responding to the environmental electric field change, and a pH-responsive polymer responding to the environmental pH change. According to the embodiment, a temperature-responsive polymer of which the stimulation from outside uses is a temperature change is used. If the temperature-responsive polymer is combined with the self-heating particles 24 to be used, the biologically active substance described below is released in response to the generation of heat of the self-heating particles 24, and thus the combination can be preferably used in a thermal therapy in which heat is applied to the affected area.

In addition, physical properties that are changed in response to stimulation are a three-dimensional structure, a melting point, a glass transition point, a state such as contraction or expansion, hydration, an electric charge, a crystal condition, and the like. The change of the physical properties is selected so as to release the biologically active substance held by the stimulation-responsive polymer to the outside by the change. For example, the three-dimensional structure of the stimulation-responsive polymer changes to a form of having many voids, or the biologically active substance is released to the outside by the contraction of the stimulation-responsive polymer. More specifically, as the physical properties, for example, properties in which the release of hydrophilic drug can be controlled in a hydration state, and the release of a hydrophobic drug can be controlled by the change of the crystal condition are preferable.

According to the embodiment, the temperature-responsive polymer is used, and, as external stimulation and physical properties changed in response to the external stimulation, for example, a polymer that contracts when temperature of an external environment is higher than the phase transition temperature (Lower Critical Solution Temperature; simply referred to as LCST), and expands when the environmental temperature is lower than the phase transition temperature is used.

As the temperature-responsive polymer, an N-alkyl-substituted acrylamide derivative polymer such as poly(N-isopropylacrylamide) (hereinafter, simply referred to as PNIPAAm) having a polyethylene main chain and an N-alkyl-substituted acrylamide side chain is included.

The PNIPAAm shows water solubility in a temperature range lower than 32°C of phase transition temperature, and rapidly becomes insoluble in a temperature range higher than the phase transition temperature and generates precipitation.

In the temperature-responsive polymer, a thermally- or photo-crosslinkable side chain may be included in the polyethylene main chain. A thermally- or photo-crosslinkable side chain of one temperature-responsive polymer and a thermally-or photo-crosslinkable side chain of another temperature-responsive polymer are cross-linked to each other, and the cross-linked portion 22 is formed, such that the polymer cross-linked body is formed.

According to the cross-linking of the side chains, the fiber 11 according to the embodiment becomes insoluble in water and an organic solvent. In addition, the outflow of the self-heating particles 24 such as magnetic particles included in the fiber can be prevented. Further, if magnetic particles are used as the self-heating particles 24, positions and directions of the fibers can be managed by a magnet using the self-heating particles 24 accumulated and included in the fiber.

In addition, if poly(N-isopropylacrylamide) (PIPAAm) of the temperature-responsive polymer is three-dimensionally cross-linked, a large amount of water can be included. Therefore, if a large amount of water is included, the temperature-responsive polymer becomes a hydrogel.

The fiber 11 according to the embodiment preferably has a polymer cross-linked body presented in Formula (1) below.

In the polymer cross-linked body, a polymer having a polyethylene main chain R₁(CH₂CH)ₗ(CH₂CH)ₘR₃, a N-alkyl-substituted acrylamide side chain CONHR₂, and a thermally- or photo-crosslinkable substituent X, and a polymer having a polyethylene main chain R₄(CH₂CH)ₛ(CH₂CH)ₜR₅, an N-allsyl-substituted acrylamide side chain CONHR₂, and a thermally- or photo-crosslinkable substituent X are cross-linked to each other by thermally- or photo-crosslinkable substituents X such that a cross-linked portion X...X is formed.

In Formula (1) below, substituents R₁, R₃, R₄, and R₅ are hydrogen atoms or linear or branched alkyl groups having 1 to 6 carbon atoms, and the substituent R₂ is an alkyl group selected from the group consisting of an isopropyl group, an n-propyl group, and a butylacrylamide. In addition, 1, m, s, and t respectively represent molar ratios (%) of monomers, and a sum of 1 and m and a sum of s and t are 100%.

The LCST of the copolymer can be controlled in a range of 5°C to 80°C by controlling the numbers of 1, m, s, and t. The LCST is preferably set to be near 40°C.

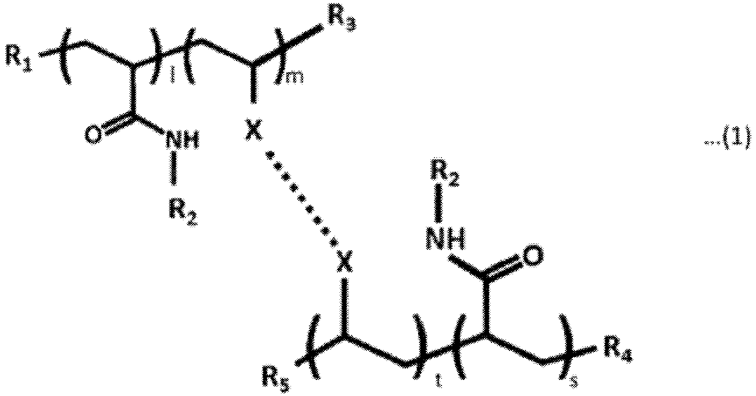

The cross-linked portion X...X is preferably represented by Formula (2) below. In Formula (2) below, n is a natural number in a range of 1 to 6. A is an NH group or a linking group of O.

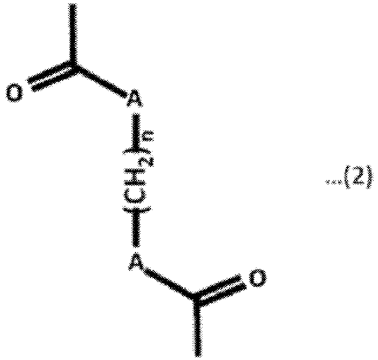

The fiber 11 according to the embodiment contains the water 23 as described above. The water 23 is contained so that a hydrogel is formed in the polymer cross-linked body and the drug particles 25 are caused to be dissolved in the water 23 and to be released, when the drug particles 25 are released as described below. According to the embodiment, as illustrated in FIG 3, the water 23 exists between molecules of the stimulation-responsive polymer 21 or in a manner of being attached to a molecule so as to be contained in the fiber 11. In addition, as a modification example of the embodiment, the fiber 11 may not contain the water 23. Particularly, if the biologically active substance is not water soluble, the fiber 11 does not need to contain the water 23.

### <Self-heating properties and biologically active substance release capabilities>

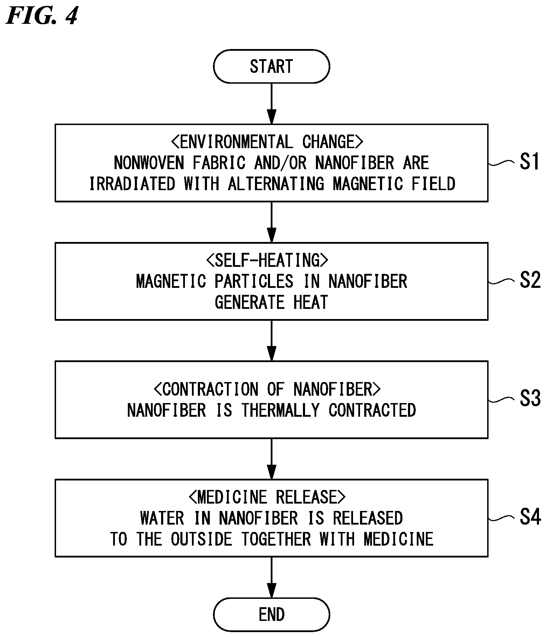

FIG. 4 is a flow chart illustrating an example of releasing the biologically active substance using the fiber according to the embodiment.

As illustrated in FIG. 4, first, the nonwoven fabric and/or the fiber according to the embodiment are irradiated with the alternating magnetic field (alternating magnetic field irradiation step S1).

According to the irradiation of the alternating magnetic field, the self-heating particles 24 (magnetic particle according to the embodiment) in the fiber generate heat (magnetic particle heat generation step S2). The fiber thermally contracts by the generation of heat by the magnetic particles (fiber thermally contraction step S3). If the fiber thermally contracts, the water 23 in the fiber is released to the outside together with the biologically active substance (the drug particles 25) (drug-releasing step S4).

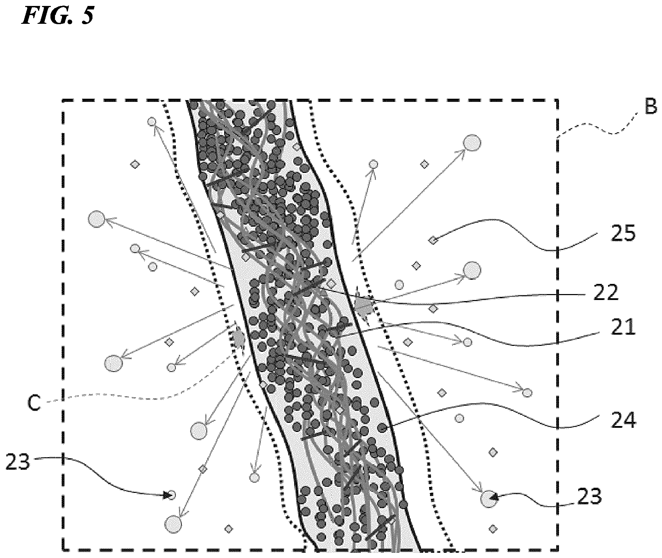

FIG. 5 is an explanation diagram illustrating an appearance of releasing a drug in a drug-releasing step S4 of FIG. 4.

As illustrated in FIG. 4, if the cross-linked body of the fiber 21 thermally contracts in the direction indicated by an arrow C by the generation of heat by the magnetic particles, the water 23 in the fiber 21 is released to the outside. In this case, the drug particles 25 are released to the outside together with the water 23.

### <Production method for fiber>

Next, the production method of the fiber according to the embodiment is described.

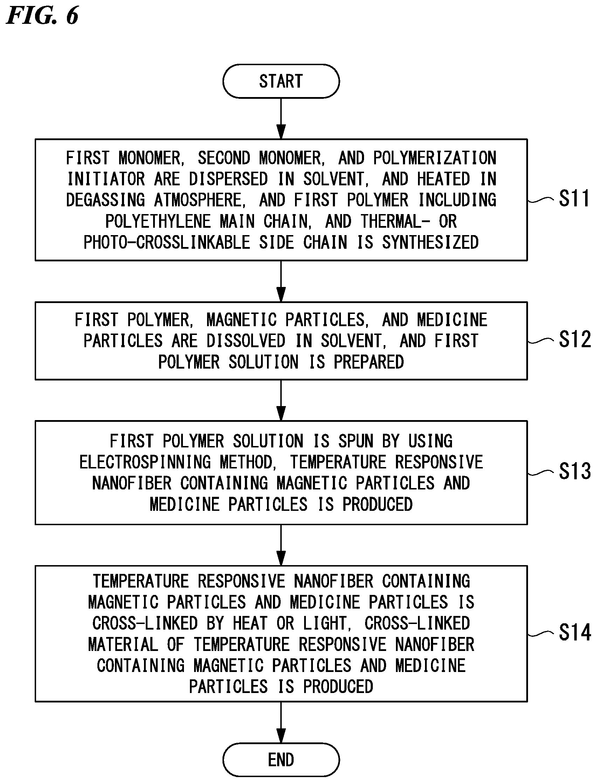

FIG. 6 is a flow chart illustrating an example of a production method for the fiber according to the embodiment.

As illustrated in FIG. 6, the production method for the fiber 11 of the embodiment includes a first polymer synthesization step S11, a first polymer solution preparation step S12, a stimulation-responsive fiber production step S13, and a stimulation-responsive fiber cross-linked body production step S14.

### <First polymer synthesization step S11>

In this step, first, a first monomer, a second monomer, and a polymerization initiator are dispersed in a solvent.

Next, copolymerization is performed by heating in a degassed atmosphere, and a first polymer having a polyethylene main chain is synthesized.

For example, the first monomer is preferably a temperature-responsive monomer, for example, having an N-alkyl-substituted acrylamide group. Accordingly, as the first polymer, a temperature-responsive polymer can be synthesized.

Specifically, the first monomer is preferably a monomer of the N-alkyl-substituted acrylamide derivative expressed by Formula (3) below.

In Formula (3) below, the substituent R₁ is a hydrogen atom or a linear or branched alkyl group having 1 to 6 carbon atoms, and the substituent R₂ is an alkyl group selected from the group consisting of an isopropyl group, an n-propyl group, and a butylacrylamide.

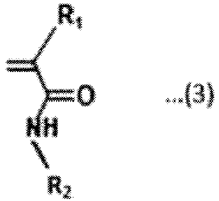

The second monomer is preferably a monomer having a thermally- or photo-crosslinkable functional group.

Specifically, the second monomer is preferably a monomer expressed by Formula (4) below. In Formula (4), the substituent R₃ is a hydrogen atom or a linear or branched alkyl group having 1 to 6 carbon atoms, and the substituent X is a thermally- or photo-crosslinkable functional group expressed by Formula (5) below. In Formula (5) below, n is a natural number in a range of 1 to 6. A is an NH group or a linking group of O.

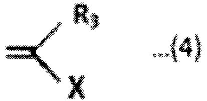

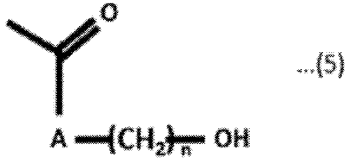

As the temperature-responsive polymer, for example, an isopropylacrylamide copolymer (hereinafter, NIPAAm copolymer) expressed by Formula (6) below is included. The substituent X is a thermally- or photo-crosslinkable functional group expressed by Formula (5) described above. In addition, 1 and m respectively represent molar ratios (%) of monomers, and a sum of 1 and m is 100%.

A molecular weight (M.W.) of the NIPAAm copolymer is preferably in a range of 1,000 to 500,000. In addition, the phase transition temperature of the NIPAAm copolymer is controlled in a range of 5°C to 80°C by adjusting the kind and the amount of the substituent X and the numbers of 1 and m.

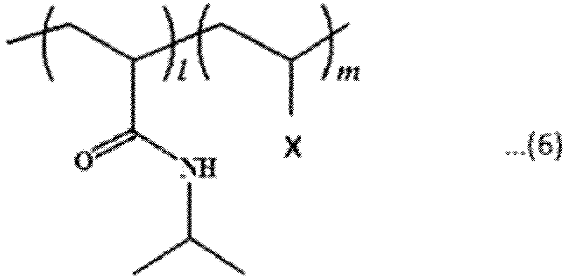

More specifically, as the NIPAAm copolymer, the NIPAAm copolymer expressed by Formula (7) below can be included.

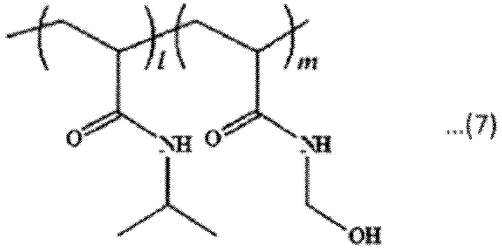

In addition, as the temperature-responsive polymer described above, aliphatic polyester expressed by Formula (7-2) can be used in addition to the above. The substituents R₁ and R₂ in the drawing are hydrogen atoms or methyl groups. In addition, n is a natural number in a range of 1 to 5. In addition, 1 and m respectively represent molar ratios (%) of monomers, and a sum of 1 and m is 100%.

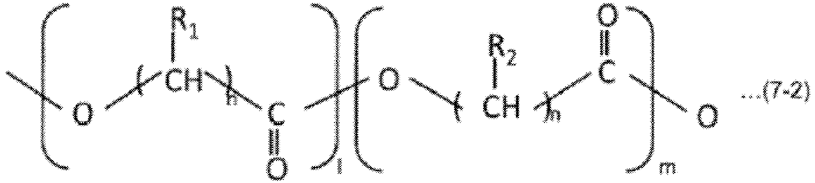

### <First polymer solution preparation step S12>

In this step, the first polymer, magnetic particles, and drug particles are dispersed in the solvent, and the first polymer solution is prepared.

When the NIPAAm copolymer is prepared as the first polymer, the NIPAAm copolymer can be dissolved in the aqueous and general-purpose organic solvent. The first polymer solution can be prepared by evenly dispersing magnetic particles and drug particles in this solvent.

### <Stimulation-responsive fiber production step S13>

In this step, the first polymer solution is spun by using the electrospinning method, and the stimulation-responsive fiber containing magnetic particles and drug particles is produced. In addition, when the stimulation-responsive fibers contain a large amount of nano-sized fibers, the stimulation-responsive fibers are called stimulation-responsive nanofibers. Further, since the stimulation-responsive fibers are fibers of which physical properties are changed in response to the temperature, in the example illustrated in FIG. 6, fibers are noted as temperature-responsive nanofibers.

The first polymer solution in which the self-heating particles 24 (magnetic particle according to the embodiment) and the biologically active substance (the drug particles 25 according to the embodiment) are evenly dispersed is used, the electrospinning method is used, and thus even fibers having diameters in a range of 50 nm to 50 µm can be processed. In this case, the diameter can be limited to a nano order by controlling conditions, and thus the fiber can be produced.

Specifically, when the first polymer solution made of the NIPAAm copolymer is used, the electrospinning method is performed under the condition of the flow velocity in a range of 0.1 mL/h to 10 mL/h, and the voltage in a range of 10 kV to 50 kV, such that the fiber of which the diameters are 50 nm or longer and shorter than 1 µm can be produced.

In addition, if the condition of the flow velocity in a range of 0.1 mL/h to 1 mL/h, and the voltage in a range of 10 kV to 30 kV is set, fibers of which the diameters are in a range of 100 nm to 800 nm can be produced.

Further, if the condition of the flow velocity in a range of 0.5 mL/h to 0.7 mL/h, and the voltage in a range of 15 kV to 20 kV is set, fibers of which the diameters are in a range of 200 nm to 500 nm can be produced.

In addition, the fibers are accumulated on the electrode surfaces under the respective conditions, and thus nonwoven fabrics in which fibers having respective diameters are formed in the mesh shape can be formed.

### <Fiber production step S14>

In this step, the stimulation-responsive fiber containing the magnetic particles and the drug particles are cross-linked by heat or light, the cross-linked body of the stimulation-responsive fiber containing the magnetic particles and the drug particles (fiber according to the embodiment) can be produced.

In the fiber production step, when the polymer having the substituent X described above is used, the fiber containing the magnetic particles and the drug particles is preferably heated under the condition of a temperature in a range of 100°C to 150°C and a period of time in a range of 10 hours to 20 hours. Accordingly, when the thermally- or photo-crosslinkable functional group X is reacted by 90% or more, the cross-linked portion 22 can be formed.

For example, the cross-linked body expressed by Formula (8) described above can be manufactured by cross-linking the NIPAAm copolymer expressed by Formula (7) described above. 1 and m respectively represent molar ratios (%) of monomers, and a sum of 1 and m is 100%.

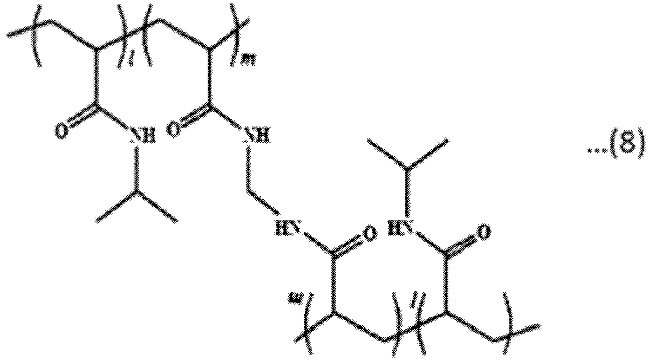

### <Behavior and effect of fiber>

### [Self-heating behavior of fiber]

In the case of the fiber including magnetic particles as the self-heating particles 24, if an alternating magnetic field is applied, the fiber can be heated to about 80°C due to hysteresis loss or the like caused by a domain wall displacement. In addition, heating temperature can be adjusted in an error range within ±1°C by adjusting the electric current or the output of the alternating magnetic field.

### [Drug-releasing behavior from fiber]

The drug particles 25 included in the fiber are released from the fiber in response to the change of the temperatures interposing the LCST of the polymer therebetween. The releasing behavior can be controlled by hydrophobicity of the drug particles 25, the molecular weight, fiber density of the fibers, and cross-linkage density.

### [Anticancer activity of fiber]

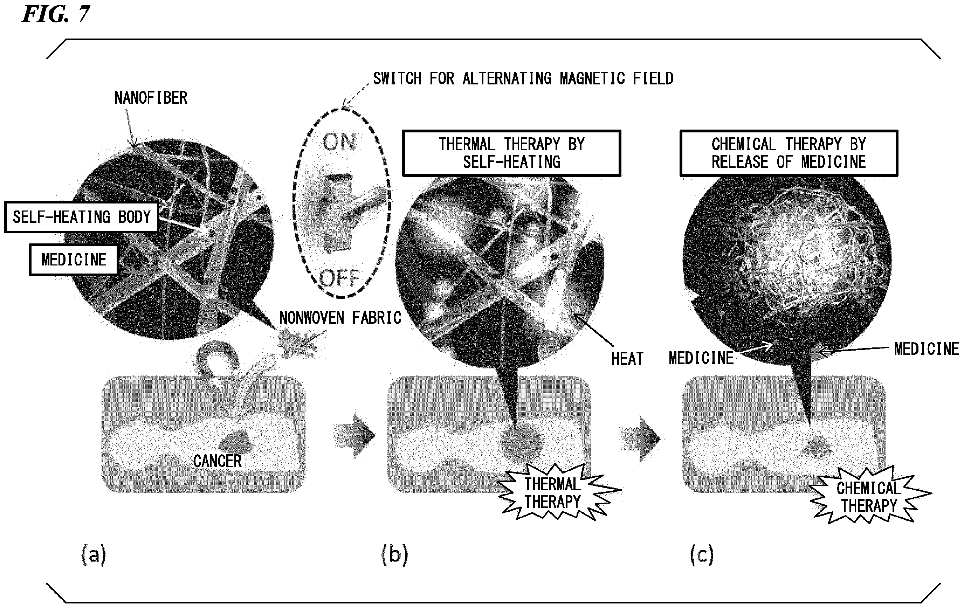

FIG. 7 is a drawing illustrating an example of a cancer treatment performed by using a nonwoven fabric according to the embodiment.

First, as illustrated in FIG. 7(a), a nonwoven fabric disposed in a certain position in the body of a human is moved to a cancer cell portion by using a magnet from outside of the body of the human. The nonwoven fabric is made of fibers including self-heating bodies (the self-heating particles 24), biologically active substances (drug particles 25) (nanofiber in the example illustrated in the drawing).

Next, an alternating magnetic field is applied. Accordingly, as illustrated in FIG. 7(b), the magnetic particles self-heat (self-heating behavior), and thermal therapy can be performed only on the cancer cell portion.

Further, as illustrated in FIG. 7(c), the self-heating fibers contract, biologically active substances (drug particle) are released (biologically active substance releasing behavior, drug-releasing behavior according to the embodiment) together with water, and chemotherapy can be performed only on the cancer cell portion.

Only the cancer cells in the affected area can be killed by the self-heating behavior and the biologically active substance releasing behavior described above.

The fiber 11 according to the embodiment is made of polymer cross-linked bodies in which the plural stimulation-responsive polymers 21 are cross-linked to each other, and has a diameter of 50 nm or longer and 1 µm or shorter, and has a configuration in which the self-heating particles 24, the drug particles 25, and the water 23 are contained between the stimulation-responsive polymer 21. Therefore, when the magnetic particles are used as the self-heating particles, after the fibers are disposed in the affected area, the affected area is irradiated with the alternating magnetic field, the inside self-heating particles self-heat, and thus the fiber can be caused to generate heat, such that the thermal therapy is applied to the affected area. In addition, if the temperature-responsive polymer is used as the stimulation-responsive polymer, the temperature-responsive fiber contracts in response to the heat generated by self-heating, the inside biologically active substance can be released to the outside together with the inside water, and thus chemotherapy by the biologically active substance can be applied to the affected area. Therefore, when the anticancer drug is used as the biologically active substance, apoptosis of the cancer cells can be promoted. In addition, if the magnetic particles are used as the self-heating particles, positions and directions of the fibers disposed in the body can be managed by using a magnet from outside. Further, the self-heating particles are stably included in the fiber, and even if the fiber contracts, the self-heating particles are not diffused to the outside together with water and the biologically active substance, such that the fiber can be safely used on the human body.

The fiber 11 according to the embodiment has a configuration in which the self-heating particles 24 are one of magnetic particles, gold nanorods, gold particles, and carbon nanotube, or the combination thereof. Therefore, the fiber 11 can self-heat due to the external environmental change such as the application of the alternating magnetic field.

The fiber 11 according to the embodiment has a configuration in which the self-heating particles 24 are magnetic particles made of iron oxide, and thus can effectively self-heat due to the application of the alternating magnetic field.

The fiber 11 according to the embodiment has a configuration in which particle diameters of the self-heating particles 24 are in a range of 10 nm to 100 nm, and thus can self-heat in a constant temperature rising rate. Therefore, when the fiber 11 is used on the affected area, the thermal therapy can be applied. In addition, the fiber can be safely used on the human body without diffusion to the outside by the contraction of the fiber. In addition, the positions and the directions of the fiber can be controlled by the magnet.

The fiber 11 according to the embodiment has a configuration in which the concentration of the self-heating particles 24 is in a range of 10 wt% to 50 wt%, and thus can be caused to self-heat at a temperature required for the thermal therapy.

The fiber 11 according to the embodiment has a configuration in which the drug particles 25 which are the biologically active substance are made of the anticancer drug, and thus the anticancer effect can be applied to the affected area.

If the fiber 11 according to the embodiment has a configuration in which the particle diameters of the drug particles 25 are 10 nm or shorter, drug in an amount capable of applying a constant drug effect to the affected area can be effectively released to the outside by the contraction of the fiber.

The fiber 11 according to the embodiment has a configuration in which the concentration of the drug particles 25 is in a range of 0.1 wt% to 10 wt%, and thus an optimum amount of a drug can be applied to the affected area.

The fiber 11 according to the embodiment has a configuration in which the stimulation-responsive polymer 21 is one selected from the group consisting of a temperature-responsive polymer, a light-responsive polymer, a magnetic field-responsive polymer, an electric field-responsive polymer, and a pH-responsive polymer, and thus can be a fiber of which physical properties and a structure can be changed in response to the external environmental change described above.

The fiber 11 according to the embodiment has a configuration in which the temperature-responsive polymer has a polyethylene main chain and an N-alkyl-substituted acrylamide side chain, and thus can be a temperature-responsive fiber that can contract or expand in response to the external temperature.

The fiber 11 according to the embodiment is a fiber having the polymer cross-linked body expressed by Formula (1) described above. The polymer cross-linked body has a configuration in which the polymer having the polyethylene main chain R₁(CH₂CH)₁(CH₂CH)ₘR₃, the N-alkyl-substituted acrylamide side chain CONHR₂, and the thermally- or photo-crosslinkable substituent X and a polymer having the polyethylene main chain R₄(CH₂CH)ₛ(CH₂CH)ₜR₅, the N-alkyl-substituted acrylamide side chain CONHR₂, and the thermally- or photo-crosslinkable substituent X are cross-linked to each other by the thermally- or photo-crosslinkable substituent X such that the cross-linked portion X...X is formed. Therefore, the fiber can include a large amount of water by a three-dimensional cross-linking structure so as to form a hydrogel, and thus the fiber can be caused to the temperature-responsive fiber that can contract and expanded in response to the external temperature.

The fiber 11 according to the embodiment has a configuration in which the cross-linked portion X...X is expressed by Formula (2) described above, and thus the three-dimensional cross-linking structure can be easily formed.

The production method for the fiber according to the embodiment includes the step S11 of synthesizing the first polymer which is the stimulation-responsive polymer including the polyethylene main chain, the stimulation-responsive side chain, and the thermally- or photo-crosslinkable side chain by dispersing the first monomer having the stimulation-responsive functional group, the second monomer having the thermally- or photo-crosslinkable functional group, and the polymerization initiator in the solvent and performing copolymerization by heat in a degassed atmosphere, the step S12 of preparing the first polymer solution by dispersing the first polymer, the self-heating particles, the drug particles in the solvent, the step S13 of producing the stimulation-responsive fiber containing the self-heating particles and the drug particles by spinning the first polymer solution by using the electrospinning method, and the step S 14 of producing the fiber made of the stimulation-responsive fiber cross-linked body containing the self-heating particles and the drug particles by cross-linking the stimulation-responsive fiber containing the self-heating particles and the drug particles by heat or light. Therefore, it is possible to easily produce a fiber that self-heats in response to the environment, that can release the drug, in which the self-heating particles are stably included in the fiber by cross-linking, and thus that can be safely used on the human body without diffusing the self-heating nanoparticles to the outside together with water and the drug particles even if the fiber contracts.

The production method of the fiber according to the embodiment has a configuration in which the self-heating particles are magnetic particles, and thus it is possible to easily produce a fiber that can self-heat by the application of the alternating magnetic field.

The production method of the fiber according to the embodiment has a configuration in which the first monomer is a monomer including a stimulation-responsive functional group, and thus it is possible to easily produce a fiber of which physical properties and a structure can be changed in response to the external environmental change.

The production method of the fiber according to the embodiment has a configuration in which the first monomer is a monomer of the N-alkyl-substituted acrylamide derivative expressed by Formula (3) described above, and thus it is possible to easily produce a fiber of which physical properties and a structure can be changed in response to the external temperature change.

The production method of the fiber according to the embodiment has a configuration in which the second monomer is the crosslinkable monomer having the thermally- or photo-crosslinkable functional group, and thus it is possible to easily produce a fiber which is insoluble in water and the organic solvent since the polymer is cross-linked.

The production method of the fiber according to the embodiment has a configuration in which the second monomer is the monomer expressed by Formula (4) described above, and thus it is possible to easily produce a fiber of which physical properties and a structure can be changed in response to the external temperature change, which is insoluble in water and the organic solvent since the polymer is cross-linked.

The production method of the fiber according to the embodiment has a configuration in which the condition of the electrospinning method is the flow velocity in a range of 0.1 mL/h to 10 mL/h, and the voltage in a range of 10 kV to 50 kV, and thus it is possible to easily produce a fiber of which the diameters are 50 nm or longer and shorter than 1 µm.

The production method of the fiber according to the embodiment has a configuration in which in the fiber production step, heating is performed under the condition of a temperature in a range of 100°C to 150°C and a period of time in a range of 10 hours to 20 hours, and thus it is possible to easily produce a fiber which is insoluble in water and the organic solvent since the polymer is cross-linked.

The nonwoven fabric according to the embodiment has a configuration in which the fibers described above are bonded in a mesh shape to form a sheet shape, and thus the shape in planar view can be easily processed according to the shape of the affected area.

In addition, according to the embodiment, the nonwoven fabric is exemplified as a subject material using the fiber as the constituent material, but the subject material using the fiber is not limited thereto. For example, a form of thread, cloth other than the nonwoven fabric, and mesh produced by weaving or bonding the fibers can be taken.

The nanofiber having self-heating properties and biologically active substance release properties according to the embodiment, the production method for the same, and the nonwoven fabric having self-heating properties and biologically active substance release capabilities are not limited to the embodiments described above, but can be achieved by being changed in various ways within the scope of the technical idea of the invention. The specific examples of the embodiments are described in the following examples, but the invention is not limited to the examples.

### Examples

### (Test Example 1)

First, NIPAAm, N-hydrosymethylacrylamide (hereinafter, simply referred to as HMAAm), and 0.01 mol% 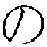 polymerization initiator azobisisobutyronitrile (simply referred to as AIBN) with respect to the NIPAAm and N-hydroxymethylacrylamide were dissolved in 20 mL of N,N-dimethylformamide (hereinafter, simply referred to as DMF).

Next, the solution was degassed and sealed in a tube and stirred at 62°C for 20 hours.

Next, precipitation in diethyl ether was performed twice, so as to refine the resultant.

In the steps above, a NIPAAm-HMAAm copolymer (referred to as P(NIPAAm-co-HMAAm): Sample in Test Example 1) was produced by the chemical reaction expressed by Formula (9) below.

P(NIPAAm-co-HMAAm) is a thermally-crosslinkable structured body.

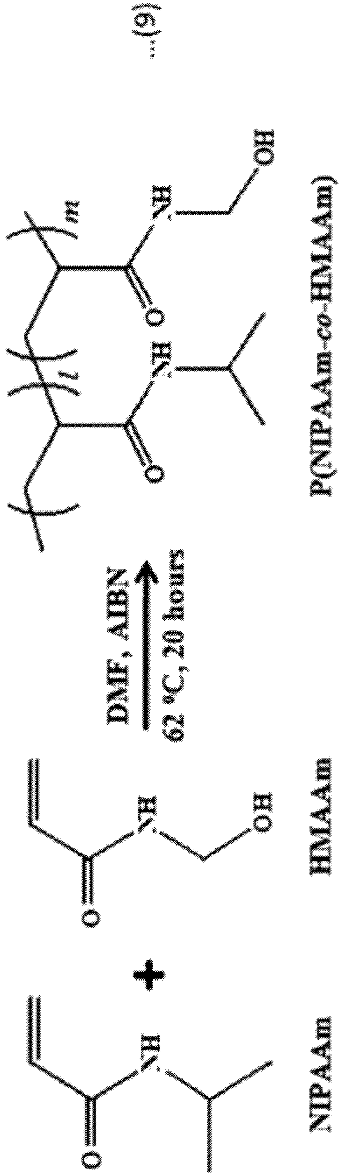

Next, a 1H-NMR spectrum measurement of the sample in Test Example 1 was performed.

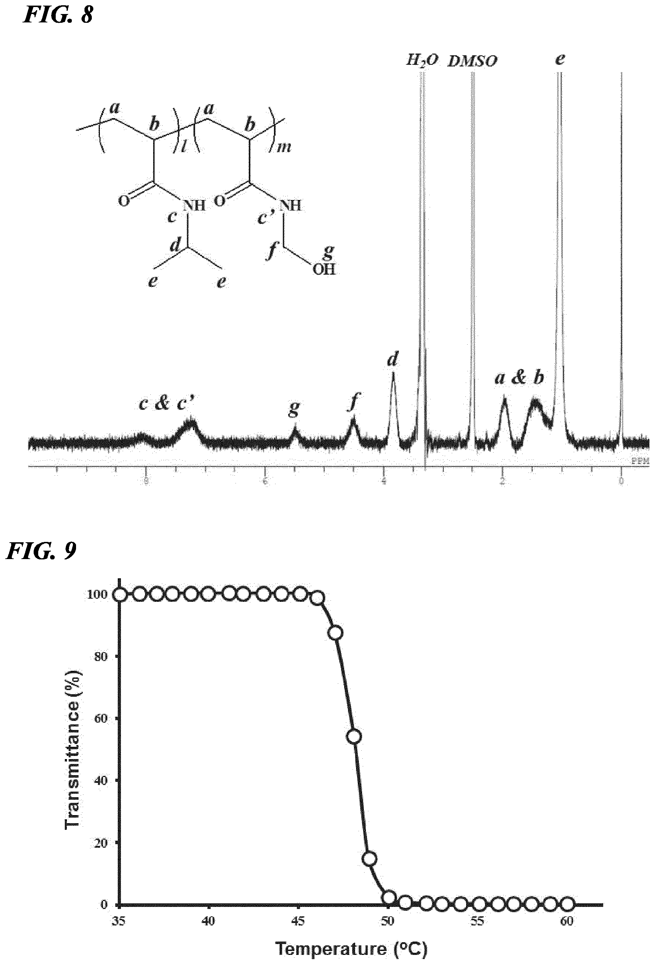

FIG. 8 is a graph illustrating the 1H-NMR spectrum measurement result of the sample in Test Example 1. Symbols corresponding to respective peak values in the chemical formula of P(NIPAAm-co-HMAAm) are also illustrated.

From the peak positions and the peak strengths in the 1H-NMR spectrum measurement result, it was found that the sample in Test Example 1 was P(NIPAAm-co-HMAAm).

Next, a change of light transparency of the sample in Test Example 1 when the environmental temperature was raised from 35°C to 60°C was examined by UV spectrum measurement.

FIG. 9 is a graph illustrating environmental temperature dependency of a UV spectrum measurement result. As illustrated in FIG. 9, in the temperature range from 47°C to 50°C, the light transmittance of the sample in Test Example 1 was drastically changed from 100% to 0%. That is, it was considered that if the temperature was caused to be higher than the scope, P(NIPAAm-co-HMAAm greatly contracts.

### (Test Example 2)

First, P(NIPAAm-co-HMAAm) (Sample in Test Example 1) was dissolved in 1,1,1,3,3,3hexafluoro-2-propanol (hereinafter, simply referred to as HFIP), so as to prepare a polymer solution.

Next, the electrospinning method was used, the voltage of 20 kV was applied at the flow velocity of 0.5 mL/h, and the polymer solution was spun such that a temperature-responsive fiber (Sample in Test Example 2) was manufactured.

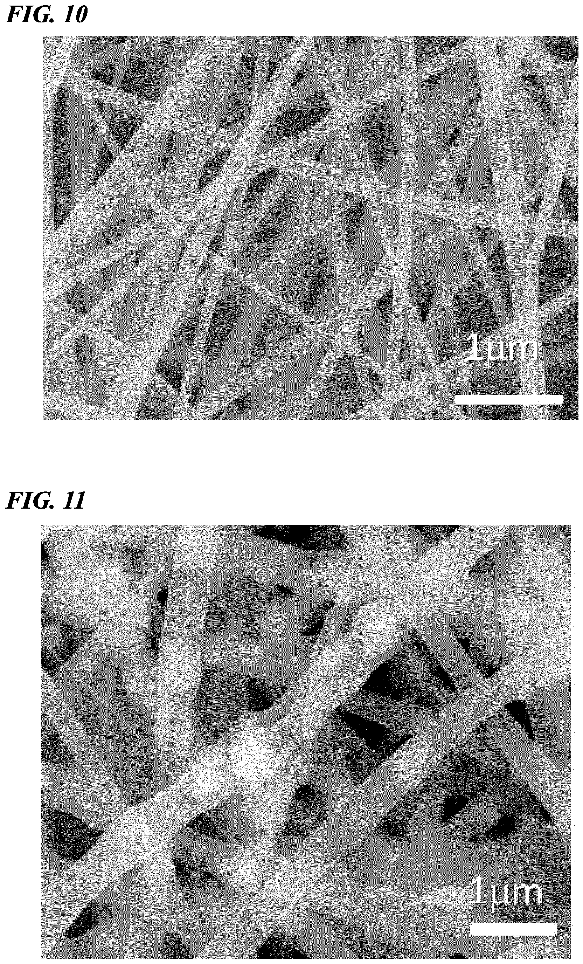

As illustrated in FIG. 10, it was found that the sample in Test Example 2 was fibers of which the average diameter was 350 nm by the observation with a scanning electron microscope (SEM).

In addition, even if the concentration of the sample in Test Example 1 was changed, fibers of which the average diameter was substantially the same could be formed.

### (Test Example 3)

P(NIPAAm-co-HMAAm) (Sample in Test Example 1), magnetic particles made of Fe₂O₃ and γ-Fe₂O₃, and drug particles made of doxorubicin which is an anticancer drug were dissolved in HFIP so that the amount of the magnetic particles was 18 wt%, so as to prepare the polymer solution.

Next, the electrospinning method was used, the voltage of 20 kV was applied at the flow velocity of 0.5 mL/h, and the polymer solution was spun such that a temperature-responsive fiber (Sample in Test Example 3) containing the magnetic particles and the drug particles was manufactured.

As illustrated in FIG. 11, it was found that the sample in Test Example 3 was fibers by the observation with the scanning electron microscope (SEM). In addition, substantially spherical particles in various sizes were formed inside.

In addition, even when the temperature-responsive fiber containing the magnetic particles and the drug particles was manufactured by changing the amount of the magnetic particles of the sample in Test Example 1 to 31 wt%, fibers of which the average diameter was substantially the same could be formed.

### (Example 1)

### <Manufacturing of temperature-responsive fiber containing magnetic particles and drug particles and having cross-linked polymer>

The temperature-responsive fiber (sample in Test Example 3) containing the magnetic particle and the drug particles was heated at 130°C for 12 hours.

Accordingly, by the chemical reaction expressed in Formula (10) below, a methylol group of HMAAm was dehydrated and condensed, and further HCHO was removed, such that the polymer of the sample in Test Example 3 was able to be thermally cross-linked.

Accordingly, the sample in Example 1 made of the temperature-responsive fiber containing the magnetic particles and the drug particles and having cross-linked polymer was manufactured.

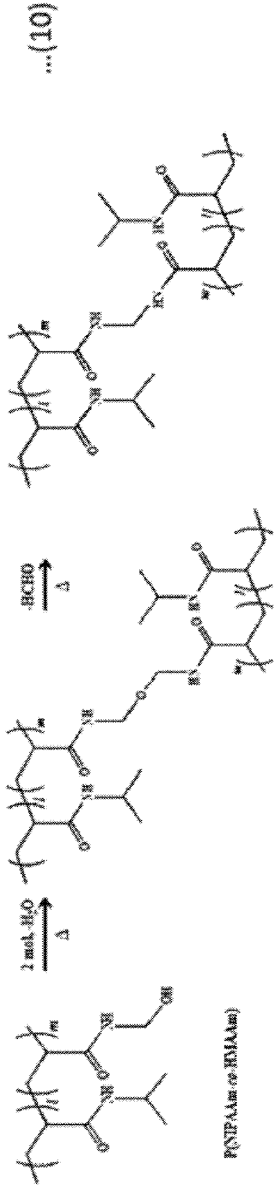

The UV spectrum measurement of the sample in Example 1 was performed.

In the UV spectrum measurement result, decrease of the peak near 305 nm could be observed. Accordingly, it was found that the polymer of the sample in Example 1 was cross-linked.

The sample in Example 1 was observed with the scanning electron microscope (SEM).

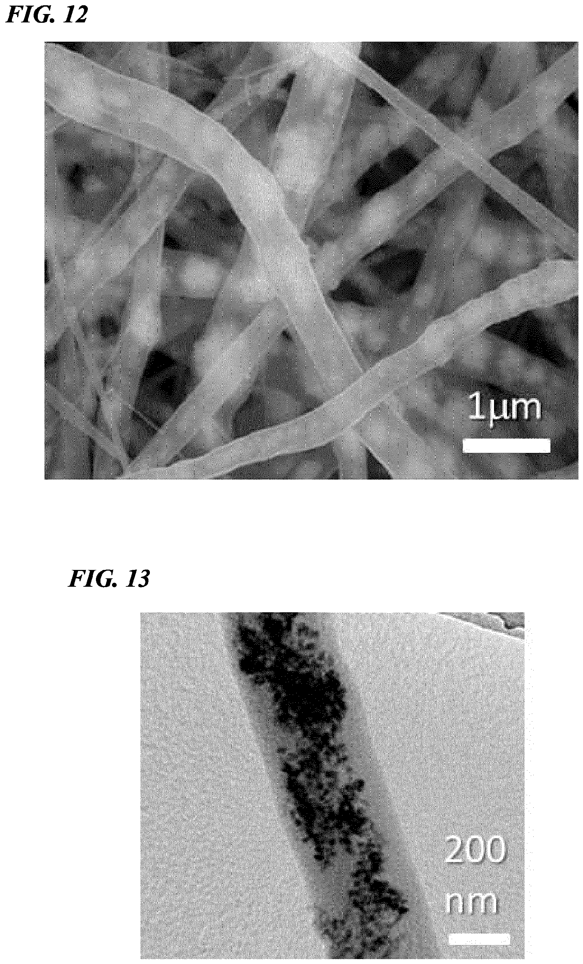

As illustrated in FIG. 12, it was found that the fiber structure was held by the sample in Example 1 by the observation with the SEM. In addition, substantially spherical particles in various sizes formed inside were maintained.

The sample in Example 1 was observed with a transmission electron microscope (TEM).

As illustrated in FIG. 13, the existence of black dot-shaped magnetic particles contained in the sample in Example 1 was found by the observation with the TEM.

The X-ray diffraction (XRD) measurement was performed on the sample in Example 1.

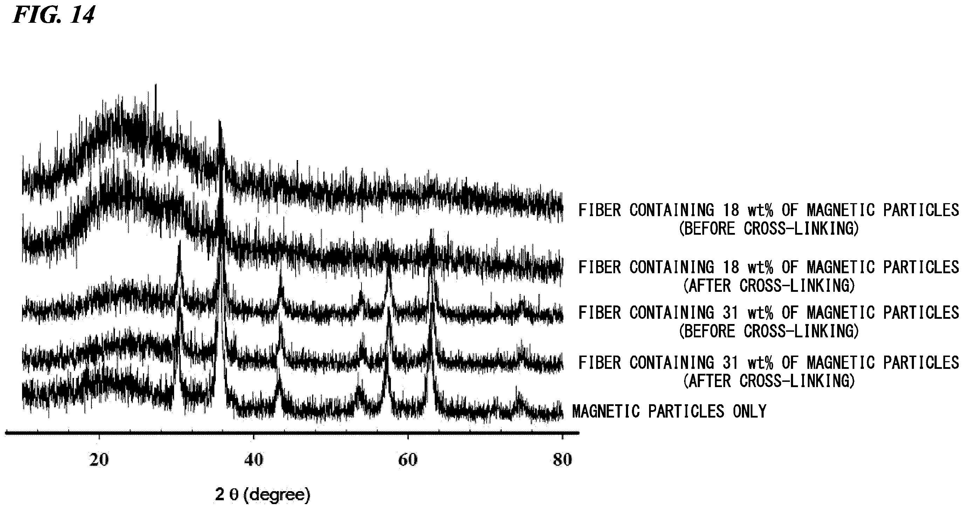

As illustrated in FIG. 14, the existence of the magnetic particles was found from the peaks of XRD.

Thermogravimetric analysis, (hereinafter, simply referred to as TGA) and energy dispersive x-ray spectroscopy (hereinafter, simply referred to as EDX) measurements were performed on the sample in Example 1.

As illustrated in Table 1, with respect to 18 wt% and 31 wt% in feed, the contents of the magnetic particles were 15 wt% and 24 wt% in the TGA measurement results, and were 11 wt% and 22 wt% in the EDX measurement results.

**[Table 1]**

| In feed(wt%) | TGA | EDX |
|---|---|---|
| 18 | 15.66±4.41 | 11.47±0.82 |
| 31 | 24.23±2.81 | 22.00±3.55 |

### <Heat generation behavior of fiber>

Heat generation behavior of the sample in Example 1 was examined.

First, 5 mg of the sample in Example 1 was taken, and was immersed in 300 µL of water in a beaker.

Next, the beaker was disposed in the winding of a copper coil (inner diameter of 5 cm, 10 turns).

Next, electric currents were caused to flow in the coil, and an alternating magnetic field of 480A, 166 kHz, 362 W was applied to the sample in Example 1 in water in the beaker.

In this manner, the relationship between the alternating magnetic field irradiation time and the sample surface temperature was examined.

In addition, the relationship between the alternating magnetic field irradiation time and the sample surface temperature was examined in the same manner as described above except that the amounts of the sample in Example 1 were set to be 15 mg and 25 mg.

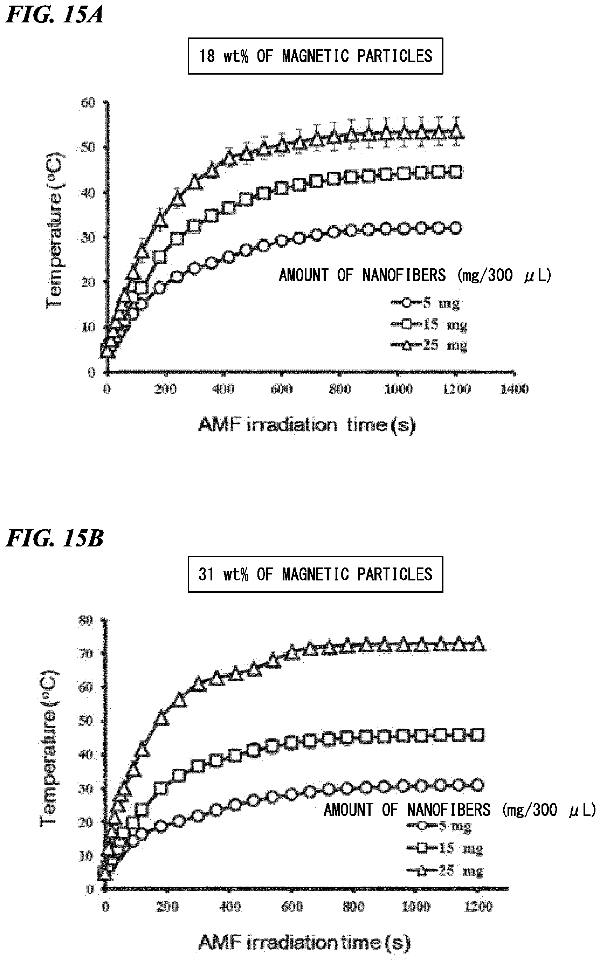

FIGS. 15A and 15B are graphs illustrating heat generation behaviors of the sample in Example 1, and are graphs illustrating relationships between alternating magnetic field irradiation times and sample surface temperatures.

According to the increase of the alternating magnetic field irradiation time, the sample surface temperature increased, and became substantially constant after 800 s. Among the samples (fiber) of Example 1 of which the amounts were 5 mg, 15 mg, and 25 mg, the sample surface temperature when the alternating magnetic field irradiation time was 800 s was highest in 25 mg of the sample, and the temperature became about 50°C.

In addition, among the sample in which an amount of the magnetic particles was 18 wt% as illustrated in FIG. 15A and the sample in which an amount of the magnetic particles was 31 wt% as illustrated in FIG. 15B, the sample surface temperature when the alternating magnetic field irradiation time was 800 s was high in the sample having 31 wt%, and the temperature became about 70°C.

That is, if the amount of the sample (fiber) in Example 1 and the content of the magnetic particles in the fiber increased, the sample surface temperature increased.

The relationship between the alternating magnetic field irradiation time and the sample surface temperature with respect to the sample in Example 1 was observed using an infrared camera.

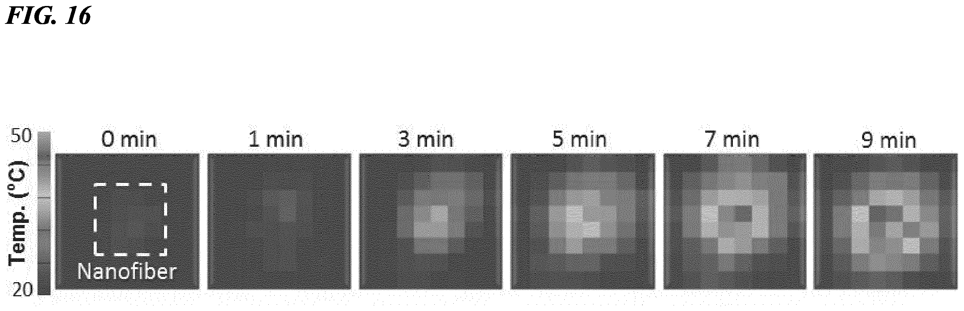

FIG. 16 is a diagram including pictures illustrating differences between alternating magnetic field irradiation times of observation images using an infrared camera.

At alternating magnetic field irradiation time of 9 (min), the fiber generated heat of about 50°C.

The relationship with the sample surface temperature to the switching of the alternating magnetic fields was examined.

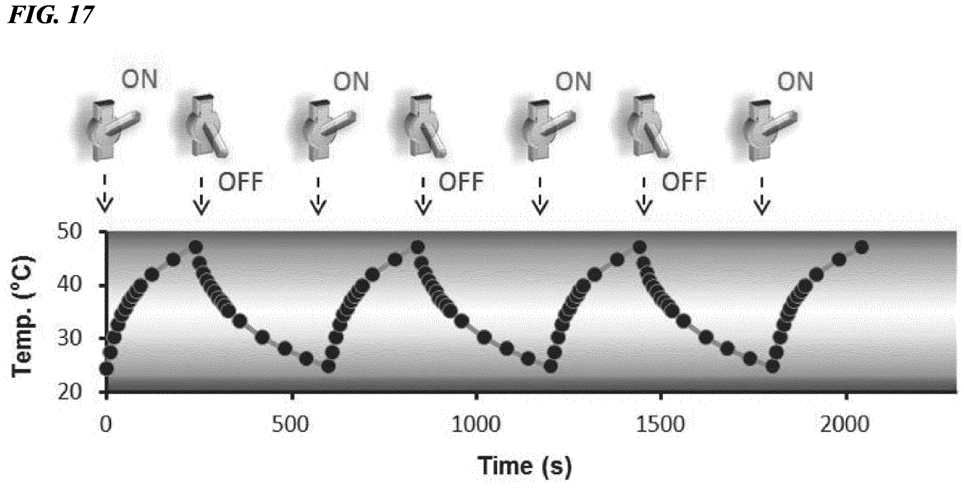

FIG. 17 is a graph illustrating a relationship of sample surface temperature to switching of alternating magnetic fields and an explanation diagram (upper diagram) thereof.

At time points corresponding to the explanation diagram (upper diagram), the ON and OFF switching of the alternating magnetic fields was repeated.

As illustrated in FIG. 17, the temperature quickly increased and decreased with respect to the ON and OFF switching of the alternating magnetic fields. From 23°C to 48°C, the reproducibility was high, and the sample surface temperature could be changed.

### <Magnetic properties of fiber>

The response of the magnetic field of the neodymium magnet of the sample in Example 1 was observed.

First, water was introduced to a petri dish having a diameter of 10 cm, and 25 mg of a sample in Example 4 was floated on the water.

Next, a neodymium magnet was brought close to the petri dish, and the appearance of the sample in Example 4 was continuously observed with a moving image.

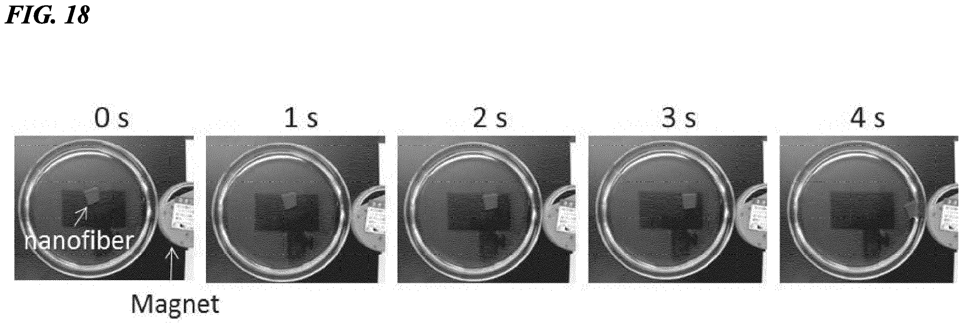

FIG. 18 is a diagram including pictures illustrating changes of positions of the sample (Nanofiber) in Example 1 according to the elapsed time when a neodymium magnet (Magnet) was brought close to the petri dish.

Pictures at elapsed times of 0 s, 1 s, 2 s, 3 s, and 4 s are illustrated. The sample in Example 4 which was floating around the center of the petri dish was quickly drawn to the neodymium magnet at 4 s.

### <Expansion and contraction behavior in response to temperature of fiber>

The behaviors of the fiber with respect to temperature changes were examined.

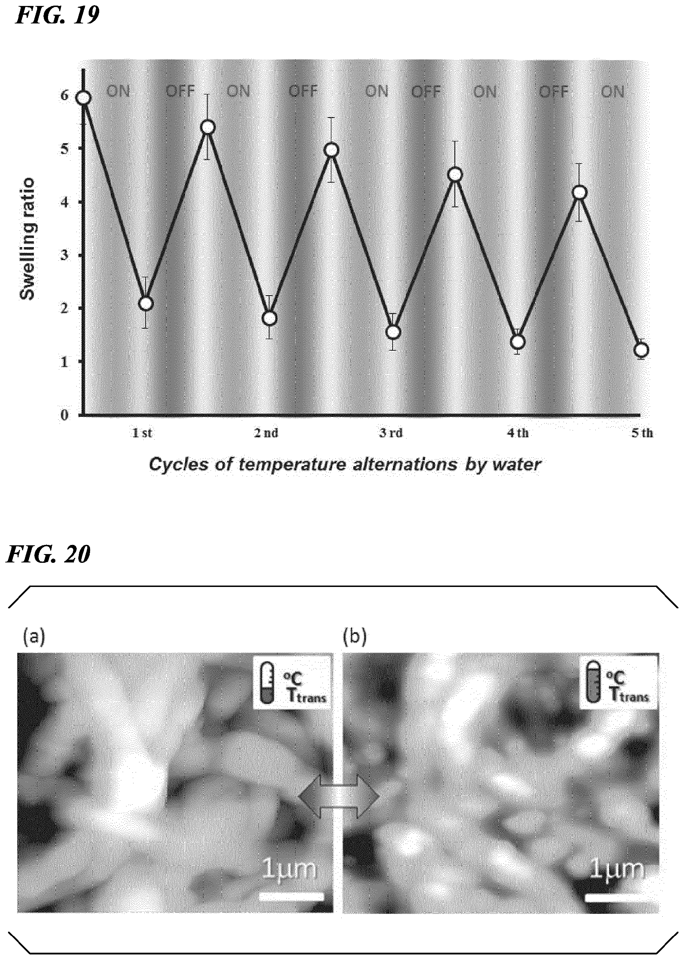

FIG. 19 is a graph illustrating expansion and contraction behavior of the sample in Example 1. With respect to the repeat of the temperature change from 20°C to 50°C, the sample in Example 1 had high reproducibility and repeated expansion and contraction changes. The expansion and contraction behaviors of the sample in Example 1 were reversible in response to the temperature.

FIG. 20 is a diagram including atomic force microscope (AFM) images of the sample (fiber) in Example 1 in water, in which FIG. 20(a) is an image at 25°C, and FIG. 20(b) is an image at 45°C. It was found that the cross-linking fiber structures were stably maintained.

### <Expansion and contraction behavior and release of drug in response to alternating magnetic field with respect to fiber>

Relationships between expansion and contraction behaviors and the release of a drug (anticancer drug) in response to the alternating magnetic field with respect to the sample (fiber) in Example 1 were examined.

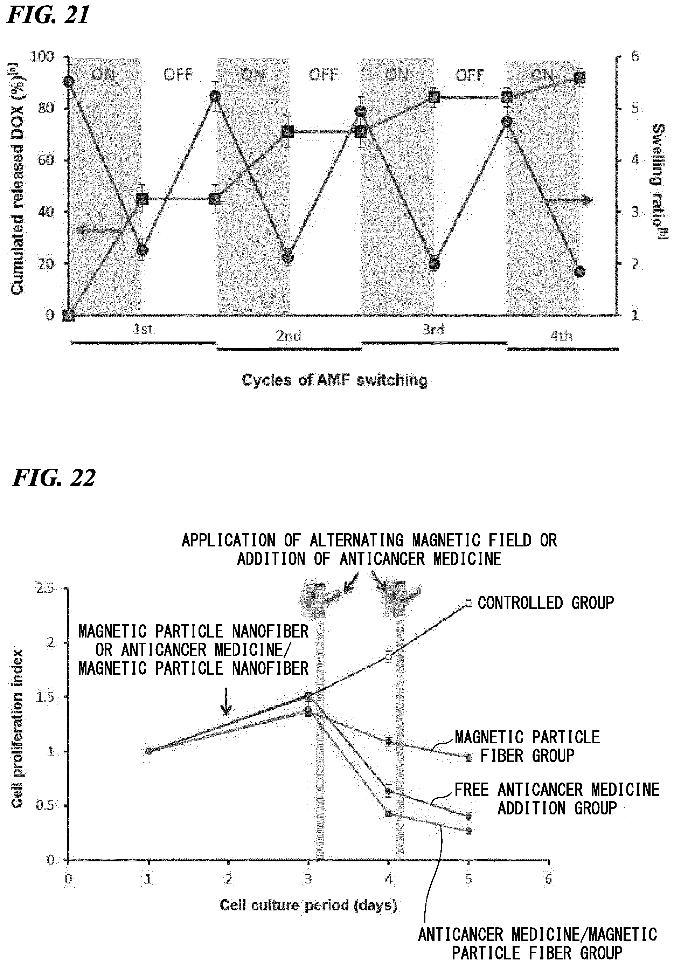

FIG. 21 is a graph illustrating relationships between expansion and contraction behaviors in response to alternating magnetic fields and release of a drug with respect to the sample (fiber) in Example 1.

First, an alternating magnetic field was applied to the sample (fiber) in Example 1. As a result, the magnetic particles contained in the sample (fiber) in Example 1 generated heat in response to the alternating magnetic field.

Next, the temperature of the sample (fiber) in Example 1 increased to the LCST or higher by the heat. As a result, the sample (fiber) in Example 1 contracted. As a result, the drug (anticancer drug) contained in the fiber was released to the outside together with water.

If the application of the alternating magnetic field was stopped, the temperature of the sample (fiber) in Example 1 was returned to the LCST or lower. As a result, the sample (fiber) in Example 1 was expanded. As a result, the release of the drug (anticancer drug) to the outside was stopped.

If the application and the stopping of the alternating magnetic field were repeated, as described above, the release and the stopping of the release of the drug (anticancer drug) to the outside were repeated.

If the application and the stopping of the alternating magnetic field were repeated four times, about 90% of a drug (anticancer drug) was released to the outside.

### <Cancer cell killing test>

An anticancer effect of the sample in Example 1 was examined by using a COLO679 cell, which is a human melanoma cell line.

First, after cells were plated at 1.0 × 10⁴ cells/well, the cells were cultured for 2 days at 37°C.

Next, the sample in Example 1 was added to the medium, and the cells were further cultured for 1 day.

Next, an alternating magnetic field (480 A, 166 kHz, 362 W) was applied for 5 minutes.

Next, the cells were cultured at 37°C for 1 day.

Next, the alternating magnetic field (480 A, 166 kHz, 362 W) was applied again, for 5 minutes.

First, a relationship between cell culture periods and cell proliferation indexes (hereinafter, simply referred to as CPI) was examined.

FIG. 22 is a graph illustrating the relationship between the cell culture periods and the cell proliferation indexes (CPI).

In a sample in which nothing was performed, as the cell culture period increased, the CPI linearly increased. That is, the number of cells increased (indicated as control group in FIG. 22).

If the sample in Example 1 was used (indicated as anticancer drug/magnetic particle fiber group in FIG. 22), after two respective time points of applying the alternating magnetic field, the CPI greatly decreased.

If a sample in which the magnetic particles only were included and the anticancer drug was not included was used (indicated as magnetic particle fiber group in FIG. 22), after two respective time points of applying the alternating magnetic field, the CPI slightly decreased.

In a reference example (indicated as free anticancer drug addition group in FIG. 22), an anticancer drug was added instead of the application of the alternating magnetic field. The same change as in the case in which the sample in Example 1 was used was brought about, but the decrease amount of the CPI was smaller than in the case in which the sample in Example 1 was used.

According to the results above, when the sample in Example 1 was used, by the anticancer drug and the self-heating, that is, by the simultaneous realization of the generation of heat and the release of the drug, more cells were killed than in the other samples, such that the proliferation of the cell was dominantly suppressed.

Next, respective samples of the control group, the magnetic particle fiber group, the free anticancer drug addition group, and the anticancer drug/magnetic particle fiber group at time points of the fifth day of the cell culture period were immunostained with DAPI, Annexin V Cy-3, and TNEL, and observed with a microscope.

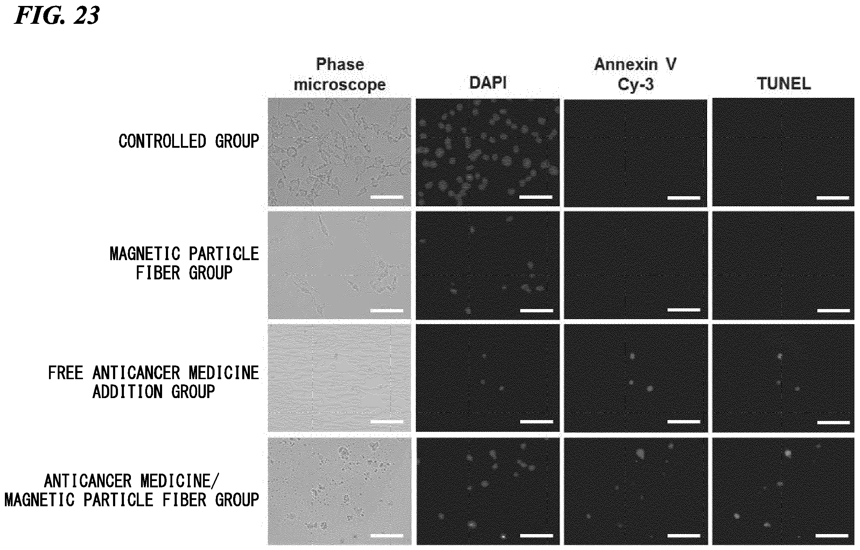

FIG. 23 is a diagram including microscope observation pictures of respective samples at time points of fifth days in cell culture period.

In FIG. 23, the left column is a phase-contrast microscope observation results of the respective samples, the second column from the left is DAPI staining results on nucleuses (indicated with blue color), the second column from the left is Annexin V staining results (indicated with red color), and the right column is TUNEL staining results (indicated with green color). White lines on the lower right sides in respective pictures are scales, and the unit of the scale is 100 mm.

In the sample in Example 1 (the anticancer drug/magnetic particle fiber group), high Annexin V staining and TUNEL staining were observed.

Since both the Annexin V and TUNEL were derived from apoptosis of cells, it was found that promotion of apoptosis of the cancer cells by the sample in Example 1 containing the anticancer drug and the magnetic particles was the cause of the proliferation suppression (killing capabilities).

In addition, apoptosis is one way by which cells configuring a multicellular organism die, and is the suicide of a cell actively performed in order to maintain an individual in a better state compared with necrosis, which is cell death caused by the deterioration of the environment inside or outside the cell, that is, programmed cell death.

### (Test Example 4)

First, ε-caprolactone (hereinafter, simply referred to as CL), D,L-lactide (hereinafter, simply referred to as DLLA), an initiator tetramethylene glycol, and a catalyst tin hexanoate were mixed, and stirred at 120°C for 24 hours. Next, precipitation in ethyl acetate was repeated two times, so as to refine the resultant. In the steps above, poly(ε-caprolactone-co-D,L-lactide) (hereinafter, simply referred to as P(CL-DLLA): sample 4) was produced.

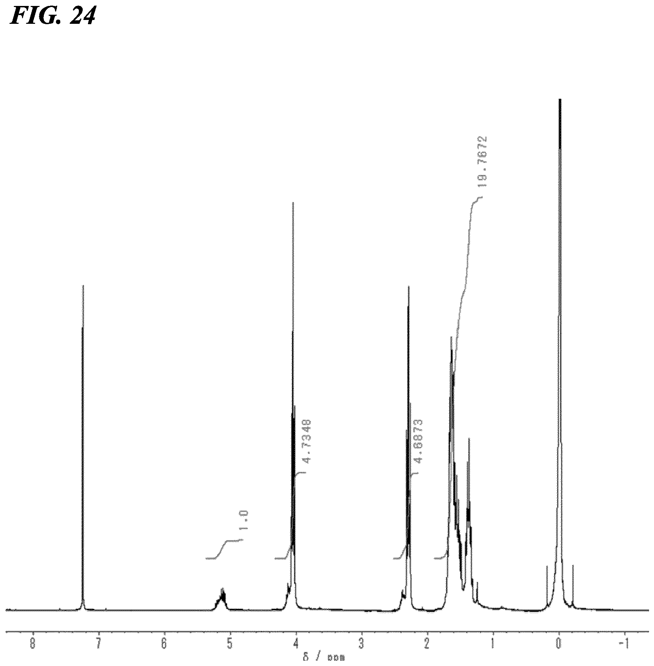

Next, a 1H-NMR spectrum measurement of the sample in Test Example 4 was performed. FIG. 24 is a graph illustrating the 1H-NMR spectrum measurement result of the sample in Test Example 4. From the peak positions and the peak strengths in the 1H-NMR spectrum measurement result, it was found that the sample in Test Example 4 was P(CL-DLLA).

### (Test Example 5)

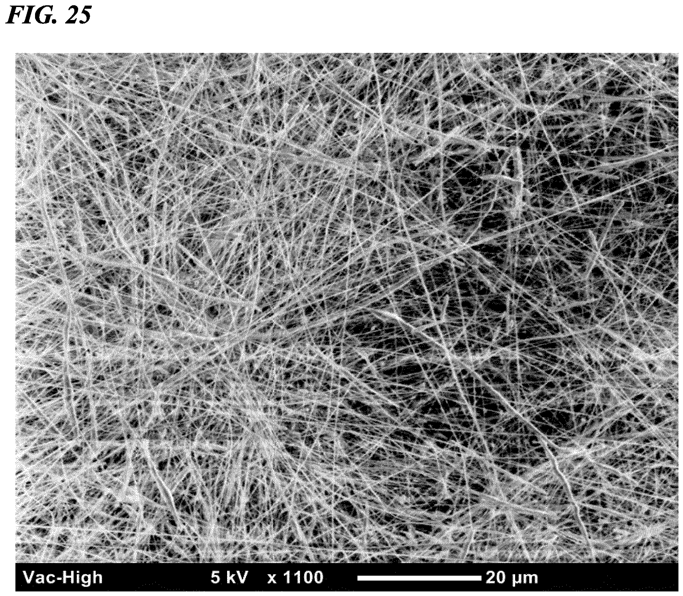

Next, simply referred to as P(CL-DLLA)), magnetic particles made of Fe₂O₃ and γ-Fe₂O₃, and drug particles made of paclitaxel, which is an anticancer drug, were dissolved in 1,1,1,3,3,3 hexafluoro-2-propanol (hereinafter, simply referred to as HFTP) so that the amount of the magnetic particles was 20 wt%, so as to prepare the polymer solution. Next, the electrospinning method was used, the voltage of 20 kV was applied at the flow velocity of 0.5 mL/h, and the polymer solution was spun such that a temperature-responsive fiber (Sample in Test Example 5) was manufactured. As illustrated in FIG. 25, it was found that the sample in Test Example 5 was fibers having the average diameter of 500 nm by the observation with the scanning electron microscope (SEM).

### <Heat generation behavior of tiber>

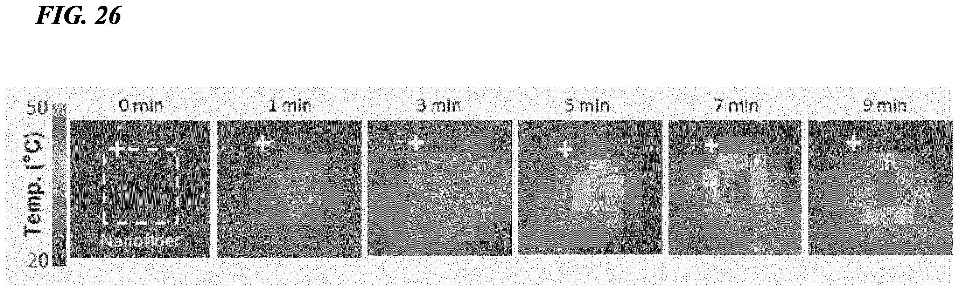

The heat generation behavior of the sample in Test Example 5 was examined. First, 5 mg of the sample in Test Example 5 was taken, and was immersed in 300 µL of water in a beaker. Next, the beaker was disposed in the winding of a copper coil (inner diameter of 5 cm, 10 turns). Next, electric currents were caused to flow in the coil, and an alternating magnetic field of 480A, 166 kHz, 362 W was applied to the sample in Test Example 5 in water in the beaker. In this manner, the relationship between the alternating magnetic field irradiation time and the sample surface temperature was observed using an infrared camera. FIG. 26 is a diagram including pictures illustrating differences between alternating magnetic field irradiation times of observation images of the infrared camera. At alternating magnetic field irradiation time of 9 (min), the fiber generated heat of about 45°C.

### Industrial Applicability

A fiber, a production method thereof, and a nonwoven fabric having self-heating properties and biologically active substance release capabilities relate to a technique used for a thermochemotherapy treatment tool for cancer that can self-heat and release a drug in response to an environmental change so as to be safely used on a human body without diffusing self-heating nanoparticles. The technique is applicable to the medical treatment tool industry such as DDS, the production industry thereof, and the like.

### Reference Signs List

1...NONWOVEN FABRIC HAVING SELF-HEATING PROPERTIES AND BIOLOGICALLY ACTIVE SUBSTANCE RELEASE CAPABILITIES, 11...FIBER, 21...STIMULATION-RESPONSIVE POLYMER, 22...CROSS-LINKED PORTION, 23...WATER, 24...SELF-HEATING PARTICLE, 25...DRUG PARTICLE

## Claims

1. A fiber having a diameter in a range of 50 nm to 50 µm, and a length of 100 times the diameter or more, the fiber comprising:
self-heating particles that generate heat in response to stimulation from outside;
a stimulation-responsive polymer of which physical properties change by directly or indirectly reacting to the stimulation; and
a biologically active substance that is held by the stimulation-responsive polymer,
wherein the biologically active substance is released to the outside in response to the changes in the physical properties of the stimulation-responsive polymer.

2. The fiber according to Claim 1, further comprising:
water.

3. The fiber according to Claim 1 or 2,
wherein the stimulation-responsive polymer includes a structure in which a plurality of polymer cross-linked bodies are cross-linked to each other.

4. The fiber according to any one of Claims 1 to 3,
wherein a diameter of the fiber is 50 nm or longer and shorter than 1 µm.

5. The fiber according to any one of Claims 1 to 4,
wherein the self-heating particles are any one of magnetic particles, gold nanorods, gold particles, and carbon nanotubes, or a combination thereof, preferably wherein the self-heating particles are magnetic particles made of iron oxide.

6. The fiber according to Claim 5,
wherein a particle diameter of the self-heating particle is in a range of 10 nm to 10 µm.

7. The fiber according to any one of Claims 1 to 6,
wherein a weight ratio of the self-heating particles is in a range of 10 wt% to 50 wt% with respect to a total weight of the fiber.

8. The fiber according to any one of Claims 1 to 7,
wherein the biologically active substance is a particle which contains an anticancer drug, preferably, wherein the particle diameter of the particles is 10 nm or shorter.

9. The fiber according to Claim 8,
wherein a weight ratio of the particles is in a range of 0.1 wt% to 10 wt% with respect to a total weight of the fiber.

10. The fiber according to any one of Claims 1 to 9,
wherein the stimulation-responsive polymer is any one selected from the group consisting of a temperature-responsive polymer, a light-responsive polymer, a magnetic field-responsive polymer, an electric field-responsive polymer, and a pH-responsive polymer.

11. The fiber according to Claim 10,
wherein the temperature-responsive polymer has a polyethylene main chain and an N-alkyl-substituted acrylamide side chain.

12. The fiber according to Claim 11, further comprising:
a polymer cross-linked body represented by Formula (1) below,
wherein, in a polymer cross-linked body, a polymer having a polyethylene main chain R₁(CH₂CH)₁(CH₂CH)ₘR₃ and an N-alkyl-substituted acrylamide side chain CONHR₂, and a thermally- or photo-crosslinkable substituent X and a polymer having a polyethylene main chain R₄(CH₂CH)ₛ(CH₂CH)ₜR₅, an N-alkyl-substituted acrylamide side chain CONHR₂, and a thermally- or photo-crosslinkable substituent X are cross-linked with the thermally- or photo-crosslinkable substituents X, so that a cross-linked portion X...X is formed as shown in Formula (1) below, wherein substituents R₁, R₃, R₄, and R₅ are hydrogen atoms or linear or branched alkyl groups having 1 to 6 carbon atoms, and a substituent R₂ is any alkyl group selected from the group consisting of an isopropyl group, an n-propyl group, and a butylacrylamide. In addition, 1, m, s, and t respectively represent molar ratios (%) of monomers, and a sum of 1 and m and a sum of s and t are 100%.

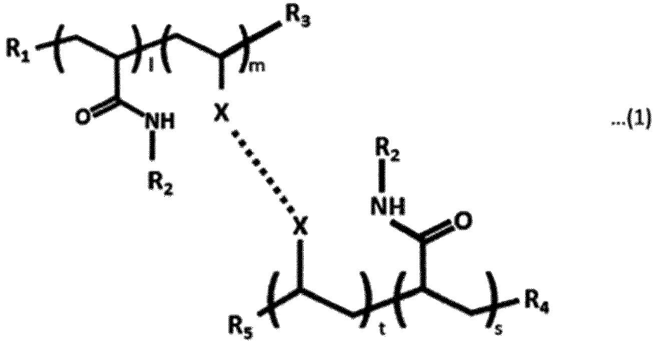

13. The fiber according to Claim 12,
wherein the cross-linked portion X...X is represented by Formula (2) below, wherein
n is a natural number in a range of 1 to 6. A is an NH group and a linking group of O.

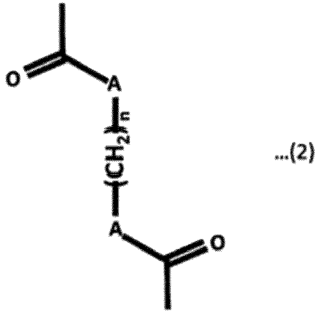

14. A production method for a fiber comprising:
a step of synthesizing a first polymer which is a stimulation-responsive polymer including a polyethylene main chain, a stimulation-responsive side chain, and a thermally- or photo-crosslinkable side chain by dispersing a first monomer having a stimulation-responsive functional group, a second monomer having a thermally- or photo-crosslinkable functional group, and a polymerization initiator in a solvent, and performing copolymerization by heat in a degassed atmosphere;
a step of preparing a first polymer solution by dispersing the first polymer, self-heating particles, preferably magnetic particles, and a biologically active substance in a solvent;
a step of producing a stimulation-responsive fiber containing the self-heating particles and the biologically active substance by spinning the first polymer solution by an electrospinning method; and
a step of producing a fiber made of a cross-linked body of the stimulation-responsive fiber containing the self-heating particles and the biologically active substance by cross-linking a stimulation-responsive fiber containing the self-heating particles and the biologically active substance by heat or light.

15. The production method for a fiber according to Claim 14,
wherein the first monomer is a monomer including the stimulation-responsive functional group.

16. The production method for a fiber according to Claim 15,
wherein the first monomer is a monomer of an N-alkyl-substituted acrylamide derivative represented by Formula (3) below, wherein an N-alkyl-substituted acrylamide group which is a stimulation-responsive functional group is included, a substituent R₁ is a hydrogen atom or a linear or branched alkyl group having 1 to 6 carbon atoms, and a substituent R₂ is any alkyl group selected from the group consisting of an isopropyl group, an n-propyl group, and a butylacrylamid.

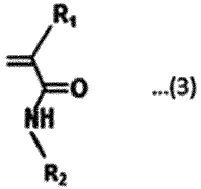

17. The production method for a fiber according to any one of Claims 14 to 16,
wherein the second monomer is a cross-linking monomer having a thermally- or photo-crosslinkable functional group, preferably,
wherein the second monomer is a monomer represented by Formula 4, wherein a substituent R₃ is a hydrogen atom or a linear or branched alkyl group having 1 to 6 carbon atoms, and a substituent X is a thermally- or photo-crosslinkable functional group represented by Formula (5) below, wherein n is a natural number in a range of 1 to 6. A is an NH group or a linking group of O.

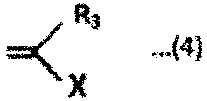

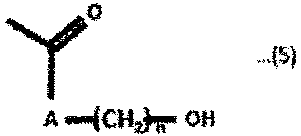

18. The production method for a fiber according to Claim 14,
wherein a condition of the electrospinning method is a flow velocity in a range of 0.1 mL/h to 10 mL/h, and a voltage in a range of 10 kV to 50 kV.

19. The production method for a fiber according to Claim 14,
wherein, in the step of producing the fiber, the heating is performed under the condition of a temperature in a range of 100°C to 150°C and a period of time in a range of 10 hours to 20 hours.

20. A nonwoven fabric,
wherein the fibers according to any one of Claims 1 to 13 are bonded to each other in a mesh shape to form a sheet shape.

## Patentansprüche

1. Faser mit einem Durchmesser in einem Bereich von 50 nm bis 50 µm und einer Länge von 100 Mal dem Durchmesser oder mehr, die Faser umfassend:
selbsterhitzende Partikel, die in Antwort auf Stimulation von außen Wärme erzeugen;
ein auf Stimulation ansprechendes Polymer, dessen physikalische Eigenschaften sich durch direktes oder indirektes Reagieren auf die Stimulation ändern; und
eine biologisch aktive Substanz, die von dem auf Stimulation ansprechenden Polymer gehalten wird,
wobei die biologisch aktive Substanz in Antwort auf die Veränderungen der physikalischen Eigenschaften des auf Stimulation ansprechenden Polymers nach außen freigesetzt wird.

2. Faser nach Anspruch 1, ferner umfassend:
Wasser.

3. Faser nach Anspruch 1 oder 2,
wobei das auf Stimulation ansprechende Polymer eine Struktur beinhaltet, in der eine Vielzahl von polymervernetzten Körpern miteinander vernetzt sind.

4. Faser nach einem der Ansprüche 1 bis 3,
wobei ein Durchmesser der Faser 50 nm oder länger und kürzer als 1 µm ist.

5. Faser nach einem der Ansprüche 1 bis 4,
wobei die selbsterhitzenden Partikel beliebige von magnetischen Partikeln, Gold-Nanostäbchen, Goldpartikeln und Kohlenstoff-Nanoröhren oder eine Kombination davon sind, bevorzugt wobei die selbsterhitzenden Partikel magnetische Partikel aus Eisenoxid sind.

6. Faser nach Anspruch 5,
wobei ein Partikeldurchmesser des selbsterhitzenden Partikels in einem Bereich von 10 nm bis 10 µm liegt.

7. Faser nach einem der Ansprüche 1 bis 6,
wobei ein Gewichtsverhältnis der selbsterhitzenden Partikel in einem Bereich von 10 Gew.-% bis 50 Gew.-% bezogen auf ein Gesamtgewicht der Faser liegt.

8. Faser nach einem der Ansprüche 1 bis 7,
wobei die biologisch aktive Substanz ein Partikel ist, das ein Antikrebsmedikament enthält, bevorzugt wobei der Partikeldurchmesser der Partikel 10 nm oder kürzer ist.

9. Faser nach Anspruch 8,
wobei ein Gewichtsverhältnis der Partikel in einem Bereich von 0,1 Gew.-% bis 10 Gew.-% bezogen auf ein Gesamtgewicht der Faser liegt.

10. Faser nach einem der Ansprüche 1 bis 9,
wobei das auf Stimulation ansprechende Polymer ein beliebiges ist, ausgewählt aus der Gruppe bestehend aus einem auf Temperatur ansprechenden Polymer, einem auf Licht ansprechenden Polymer, einem auf Magnetfeld ansprechenden Polymer, einem auf elektrisches Feld ansprechenden Polymer und einem auf pH ansprechenden Polymer.

11. Faser nach Anspruch 10,
wobei das auf Temperatur ansprechende Polymer eine Polyethylen-Hauptkette und eine N-Alkyl-substituierte Acrylamid-Seitenkette hat.

12. Faser nach Anspruch 11, ferner umfassend:
einen polymervernetzten Körper, dargestellt durch Formel (1) unten, wobei in einem polymervernetzten Körper ein Polymer mit einer Polyethylen-Hauptkette R₁(CH₂CH)₁(CH₂CH)ₘR₃ und einer N-Alkyl-substituierten Acrylamid-Seitenkette CONHR₂, und ein thermisch oder photovernetzbarer Substituent X und ein Polymer mit einer Polyethylen-Hauptkette R₄(CH₂CH)s(CH₂CH)ₜR₅, einer N-Alkyl-substituierten Acrylamid-Seitenkette CONHR₂ und einem thermisch oder photovernetzbaren Substituenten X mit den thermisch oder photovernetzbaren Substituenten vernetzt werden, sodass ein vernetzter Abschnitt X...X gebildet wird, wie in Formel (1) unten gezeigt, wobei
Substituenten R₁, R₃, R₄ und R₅ Wasserstoffatome oder lineare oder verzweigte Alkylgruppen mit 1 bis 6 Kohlenstoffatomen sind und ein Substituent R₂ irgendeine Alkylgruppe ist, ausgewählt aus der Gruppe bestehend aus einer Isopropylgruppe, einer n-Propylgruppe und einem Butylacrylamid. Außerdem stellen 1, m, s bzw. t jeweils Molverhältnisse (%) von Monomeren dar, und eine Summe von 1 und m und eine Summe von s und t sind 100 %.

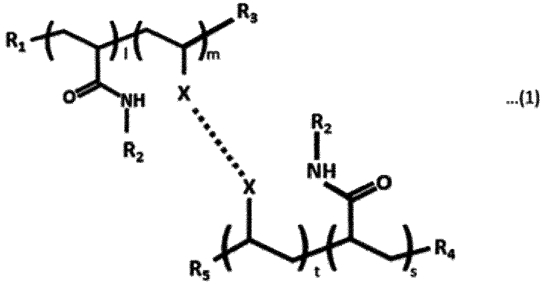

13. Faser nach Anspruch 12,
wobei der vernetzte Abschnitt X...X dargestellt wird durch Formel (2) unten, wobei n eine natürliche Zahl in einem Bereich von 1 bis 6 ist. A ist eine NH-Gruppe und eine Verbindungsgruppe von O.

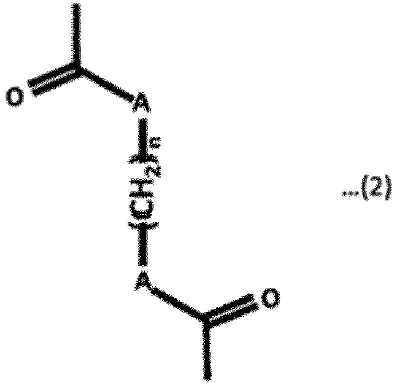

14. Herstellungsverfahren für eine Faser, umfassend:
einen Schritt vom Synthetisieren eines ersten Polymers, welches ein auf Stimulation ansprechendes Polymer ist, einschließlich einer Polyethylen-Hauptkette, einer auf Stimulation ansprechenden Seitenkette und einer thermisch- oder photovernetzbaren Seitenkette, durch Dispergieren eines ersten Monomers mit einer auf Stimulation ansprechenden funktionalen Gruppe, eines zweiten Monomers mit einer thermisch oder photovernetzbaren funktionalen Gruppe und eines Polymerisationsinitiators in einem Lösungsmittel und Durchführen von Copolymerisation durch Hitze in einer entgasten Atmosphäre;
einen Schritt vom Herstellen einer ersten Polymerlösung durch Dispergieren des ersten Polymers, selbsterhitzender Partikel, bevorzugt magnetischer Partikel, und einer biologisch aktiven Substanz in einem Lösungsmittel;
einen Schritt vom Herstellen einer auf Stimulation ansprechenden Faser, enthaltend die selbsterhitzenden Partikel und die biologisch aktive Substanz durch Spinnen der ersten Polymerlösung mittels eines Elektrospinnverfahrens; und
einen Schritt vom Herstellen einer Faser, hergestellt aus einem vernetzten Körper der auf Stimulation ansprechenden Faser, enthaltend die selbsterhitzenden Partikel und die biologische aktive Substanz, durch Vernetzen einer auf Stimulation ansprechenden Faser, enthaltend die selbsterhitzenden Partikel und die biologisch aktive Substanz, durch Hitze oder Licht.

15. Herstellungsverfahren für eine Faser nach Anspruch 14, wobei das erste Monomer ein Monomer einschließlich der auf Stimulation ansprechenden funktionalen Gruppe ist.

16. Herstellungsverfahren für eine Faser nach Anspruch 15, wobei das erste Monomer ein Monomer eines N-Alkyl-substituierten Acrylamidderivats ist, dargestellt durch Formel (3) unten, wobei eine N-Alkyl-substituierte Acrylamidgruppe, die eine auf Stimulation ansprechende funktionale Gruppe ist, eingeschlossen ist, ein Substituent R₁ ein Wasserstoffatom oder eine lineare oder verzweigte Alkylgruppe mit 1 bis 6 Kohlenstoffatomen ist und ein Substituent R₂ irgendeine Alkylgruppe ist, ausgewählt aus der Gruppe bestehend aus einer Isopropylgruppe, einer n-Propylgruppe und einem Butylacrylamid.

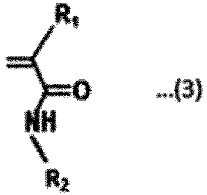

17. Herstellungsverfahren für eine Faser nach einem der Ansprüche 14 bis 16,
wobei das zweite Monomer ein vernetzendes Monomer mit einer thermisch- oder photovernetzbaren funktionalen Gruppe ist, bevorzugt
wobei das zweite Monomer ein Monomer ist, dargestellt durch Formel (4), wobei ein Substituent R₃ ein Wasserstoffatom oder eine lineare oder verzweigte Gruppe mit 1 bis 6 Kohlenstoffatomen ist und ein Substituent X eine thermisch- oder photovernetzbare funktionale Gruppe ist, dargestellt durch Formel (5) unten, wobei n eine natürliche Zahl in einem Bereich von 1 bis 6 ist.
A ist eine NH-Gruppe oder eine Verbindungsgruppe von O.

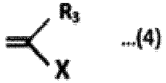

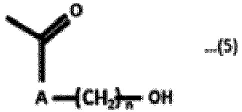

18. Herstellungsverfahren für eine Faser nach Anspruch 14, wobei eine Bedingung des Elektrospinnverfahrens eine Fließgeschwindigkeit in einem Bereich von 0,1 mL/h bis 10 mL/h und eine Spannung in einem Bereich von 10 kV bis 50 kV ist.

19. Herstellungsverfahren für eine Faser nach Anspruch 14, wobei in dem Schritt der Herstellung der Faser das Erhitzen unter der Bedingung von einer Temperatur in einem Bereich von 100°C bis 150°C und einem Zeitraum in einem Bereich von 10 Stunden bis 20 Stunden durchgeführt wird.

20. Faservlies,
wobei die Fasern nach einem der Ansprüche 1 bis 13 in einer Maschenform aneinander gebunden sind, um eine Schichtform zu bilden.

## Revendications

1. Fibre présentant un diamètre compris dans l'intervalle allant de 50 nm à 50 µm, et une longueur de 100 fois son diamètre ou plus, laquelle fibre comprend :
- des particules auto-thermogènes, qui génèrent de la chaleur en réponse à une stimulation venant de l'extérieur,
- un polymère sensible à une stimulation, dont les propriétés physiques changent en réaction directe ou indirecte à une stimulation,
- et une substance dotée d'une activité biologique, qui est retenue par le polymère sensible à une stimulation,
laquelle substance dotée d'une activité biologique est libérée vers l'extérieur en réponse aux changements des propriétés physiques du polymère sensible à une stimulation.

2. Fibre conforme à la revendication 1, qui comprend en outre de l'eau.

3. Fibre conforme à la revendication 1 ou 2, dans laquelle le polymère sensible à une stimulation inclut une structure dans laquelle plusieurs corps réticulés de polymère sont liés les uns aux autres par réticulation.

4. Fibre conforme à l'une des revendications 1 à 3, de laquelle fibre le diamètre vaut 50 nm ou plus, mais moins de 1 µm.

5. Fibre conforme à l'une des revendications 1 à 4, dans laquelle les particules auto-thermogènes sont des particules de n'importe lequel des types suivants : particules magnétiques, nano-barres d'or, particules d'or, et nanotubes de carbone, ou une combinaison de telles particules, étant entendu que ces particules auto-thermogènes sont de préférence des particules magnétiques faites d'oxyde de fer.

6. Fibre conforme à la revendication 5, dans laquelle le diamètre de particule des particules auto-thermogènes vaut de 10 nm à 10 µm.

7. Fibre conforme à l'une des revendications 1 à 6, dans laquelle la proportion pondérale des particules auto-thermogènes vaut de 10 % à 50 %, en poids rapporté au poids total de la fibre.

8. Fibre conforme à l'une des revendications 1 à 7, dans laquelle la substance dotée d'une activité biologique est constituée de particules contenant un médicament anti-cancéreux, lesquelles particules présentent de préférence un diamètre de particule de 10 nm ou moins.

9. Fibre conforme à la revendication 8, dans laquelle la proportion pondérale des particules vaut de 0,1 % à 10 %, en poids rapporté au poids total de la fibre.

10. Fibre conforme à l'une des revendications 1 à 9, dans laquelle le polymère sensible à une stimulation est un polymère choisi dans l'ensemble constitué par un polymère sensible à la température, un polymère sensible à la lumière, un polymère sensible à un champ magnétique, un polymère sensible à un champ électrique, et un polymère sensible au pH.

11. Fibre conforme à la revendication 10, dans laquelle le polymère sensible à la température comporte une chaîne principale de type polyéthylène et une chaîne latérale de type N-alkyl-acrylamide.

12. Fibre conforme à la revendication 11, qui comprend en outre un corps polymère réticulé, représenté par la formule (1) donnée ci-dessous, dans lequel corps polymère réticulé un polymère comportant une chaîne principale de type polyéthylène, symbolisée par R₁(CH₂CH)₁(CH₂CH)ₘR₃, une chaîne latérale de type N-alkyl-acrylamide, symbolisée par CONHR₂, et un substituant pour thermo-réticulation ou photo-réticulation, symbolisé par X, et un polymère comportant une chaîne principale de type polyéthylène, symbolisée par R₄(CH₂CH)ₛ(CH₂CH)ₜR₅, une chaîne latérale de type N-alkyl-acrylamide, symbolisée par CONHR₂, et un substituant pour thermo-réticulation ou photo-réticulation, symbolisé par X, sont réticulés, par l'intermédiaire des substituants X pour thermo-réticulation ou photo-réticulation, de manière à ce qu'il se forme un raccord de réticulation symbolisé par X......X,
étant entendu que dans la formule (1) donnée ci-dessous, les symboles R₁, R₃, R₄ et R₅ représentent des atomes d'hydrogène ou des groupes alkyle à chaîne linéaire ou ramifiée, comportant de 1 à 6 atomes de carbone, et le symbole R₂ représente un groupe alkyle choisi dans l'ensemble formé par les groupes isopropyle, n-propyle et butyle, et que les indices 1, m, s et t correspondent aux rapports molaires, en pourcentages, des monomères respectifs, les sommes 1 + m et s + t valant chacune 100 %.

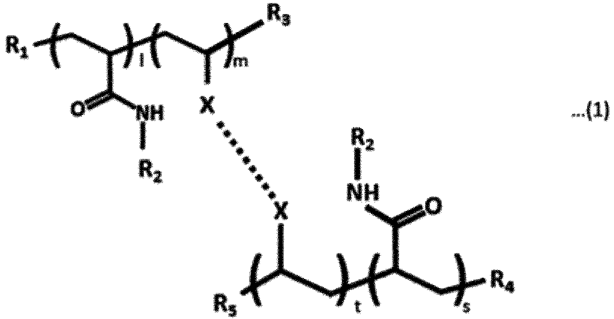

13. Fibre conforme à la revendication 12, dans laquelle le raccord de réticulation symbolisé par X......X est représenté par la formule (2) ci-dessous, dans laquelle formule (2) l'indice n est un nombre entier valant de 1 à 6, et A représente un chaînon symbolisé par NH ou O.

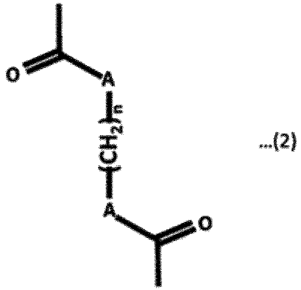

14. Procédé de production d'une fibre, qui comporte :
- une étape consistant à synthétiser un premier polymère qui est un polymère sensible à une stimulation, comprenant une chaîne principale de type polyéthylène, une chaîne latérale sensible à une stimulation et une chaîne latérale pour thermo-réticulation ou photo-réticulation, en dispersant, dans un solvant, un premier monomère comportant un groupe fonctionnel sensible à une stimulation, un deuxième monomère comportant un groupe fonctionnel pour thermo-réticulation ou photo-réticulation, et un amorceur de polymérisation, et en les faisant copolymériser, à chaud et à l'abri de l'air,
- une étape consistant à préparer une première solution de polymère en dispersant, dans un solvant, le premier polymère, des particules auto-thermogènes, qui sont de préférence des particules magnétiques, et une substance dotée d'une activité biologique,
- une étape consistant à produire une fibre sensible à une stimulation, contenant les particules auto-thermogènes et la substance dotée d'une activité biologique, en soumettant la première solution de polymère à un procédé d'électrofilage,
- et une étape consistant à produire une fibre faite d'un corps polymère réticulé, à base de cette fibre sensible à une stimulation, contenant les particules auto-thermogènes et la substance dotée d'une activité biologique, en faisant réticuler, sous l'action de la chaleur ou de la lumière, cette fibre sensible à une stimulation, contenant les particules auto-thermogènes et la substance dotée d'une activité biologique.

15. Procédé de production d'une fibre, conforme à la revendication 14, dans lequel le premier monomère est un monomère comportant le groupe fonctionnel sensible à une stimulation.

16. Procédé de production d'une fibre, conforme à la revendication 15, dans lequel le premier monomère est un monomère dérivé d'un N-alkyl-acrylamide et représenté par la formule (3) ci-dessous, dans laquelle formule (3) est figuré un groupe N-alkyl-acrylamido qui est un groupe fonctionnel sensible à une stimulation, le symbole R₁ représente un atome d'hydrogène ou un groupe alkyle à chaîne linéaire ou ramifiée, comportant de 1 à 6 atomes de carbone, et le symbole R₂ représente un groupe alkyle choisi dans l'ensemble formé par les groupes isopropyle, n-propyle et butyle.

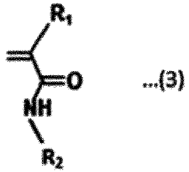

17. Procédé de production d'une fibre, conforme à l'une des revendications 14 à 16, dans lequel le deuxième monomère est un monomère pour réticulation qui comporte un groupe fonctionnel pour thermo-réticulation ou photo-réticulation, et dans lequel, de préférence, le deuxième monomère est un monomère représenté par la formule (4) donnée ci-dessous, dans laquelle formule (4) le symbole R₃ représente un atome d'hydrogène ou un groupe alkyle à chaîne linéaire ou ramifiée, comportant de 1 à 6 atomes de carbone, et le symbole X représente un groupe fonctionnel pour thermo-réticulation ou photo-réticulation, ce groupe étant représenté par la formule (5) donnée ci-dessous, dans laquelle l'indice n est nombre entier valant de 1 à 6 et A représente un chaînon symbolisé par NH ou O.

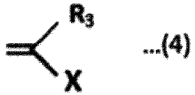

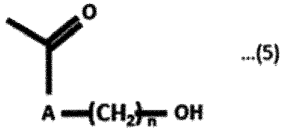

18. Procédé de production d'une fibre, conforme à la revendication 14, dans lequel les conditions opératoires de l'électrofilage sont un débit de 0,1 à 10 mL/h et une tension de 10 à 50 kV.

19. Procédé de production d'une fibre, conforme à la revendication 14, dans lequel, dans l'étape de production de la fibre, le chauffage est mené à une température de 100 à 150 °C, durant un laps de temps de 10 à 20 heures.

20. Textile non-tissé, dans lequel des fibres conformes à l'une des revendications 1 à 13 sont attachées les unes aux autres à la façon d'un tamis, de manière à former une feuille.
